(19)

**Europäisches Patentamt**

**European Patent Office**

**Office européen des brevets**

(11)     **EP 3 662 756 A1**

(12)     **EUROPEAN PATENT APPLICATION**

(43) Date of publication:
**10.06.2020  Bulletin 2020/24**

(21) Application number: **18306648.9**

(22) Date of filing: **07.12.2018**

(51) Int Cl.:
*A23D 7/015* (2006.01)       *A23C 9/12* (2006.01)
*A23G 9/40* (2006.01)        *A23L 35/00* (2016.01)
*A61K 8/06* (2006.01)        *B01F 3/08* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **General Mills, Inc.**
  **Minneapolis, MN 55426 (US)**
• **Université de Bordeaux**
  **33000 Bordeaux (FR)**
• **CENTRE NATIONAL DE LA RECHERCHE SCIENTIFIQUE**
  **75016 Paris (FR)**

• **Institut Polytechnique de Bordeaux**
  **33400 Talence (FR)**

(72) Inventors:
• **GOIBIER, Lucie**
  **34350 Valras Plage (FR)**
• **FAURE, Chrystel**
  **33000 BORDEAUX (FR)**
• **LEAL CALDERON, Fernando**
  **33650 LA BREDE (FR)**
• **CHAUDEMANCHE, Cyril**
  **38200 Serpaize (FR)**

(74) Representative: **Lavoix**
  **2, place d'Estienne d'Orves**
  **75441 Paris Cedex 09 (FR)**

(54)     **MULTIPLE EMULSIONS, METHOD OF MAKING THEM AND APPLICATIONS IN FOOD, COSMETICS AND PHARMACEUTICALS**

(57)     The present invention relates to a multiple emulsion comprising or consisting of P1/O/W2, wherein P1 is an aqueous phase forming droplets or a gas phase forming bubbles, said droplets or bubbles being dispersed in O thereby forming P1/O, wherein O is an oily phase comprising crystals, wherein W2 is an aqueous phase comprising at least one hydrophilic surfactant, wherein P1/O globules are formed in W2, wherein said oily phase O comprises at least 90%, preferably at least 92%, and even preferably at least 95%, by mass of triglycerides with respect to the mass of the O phase.

The present invention relates to a process for preparing such multiple emulsion and applications thereof. Food, cosmetic and pharmaceutical compositions as well as a packaging containing a composition are claimed.

EP 3 662 756 A1

## Description

The present invention concerns multiple emulsions, and in particular double emulsions.

The present invention concerns multiple emulsions for use in a food, a cosmetic or a pharmaceutical composition.

The present invention concerns a process for preparing such multiple emulsions and compositions containing same.

## Technological background

Multiple emulsions, in particular water-in-oil-in-water (W1/O/W2), are complex liquid dispersions of oil globules containing small aqueous droplets and are of significant interest for many industrial applications like foods, pharmaceutics or cosmetics. Formulations with reduced number of additives and/or limited additive concentrations are highly sought after.

W1/O/W2 have a great potential for industrial applications such as pharmaceuticals, or cosmetics as the internal aqueous phase enables the entrapment of hydrophilic compounds. Double emulsions can thus be used for drug encapsulation and isolation, controlled release, and targeted delivery. Many applications can also be considered in the food area. Multiple emulsions of the W1/O/W2 type can be used for entrapment and controlled release of aroma and flavor, protection of probiotics, taste masking and for lowering the fat content in foods by including an aqueous fraction in the oil globules.

Long-term kinetic stability is required for most of the practical applications. Double emulsions are generally prepared with two emulsifiers of opposite solubility (water-soluble (hydrophilic) and oil-soluble (lipophilic)). Both emulsifiers mix at the interfaces and the stability of the films with respect to coalescence is governed by the composition of the binary mixture. Coalescence can occur: i) between small inner droplets, ii) between oil globules and iii) between the external phase and the inner droplets dispersed within the globules.

Initially, the inner aqueous droplets contain an encapsulated compound (EC), and the external phase comprises an osmotic regulator (OR). Because of the concentration gradients, both solutes tend to be transferred from one compartment to the other across the oil phase. Water transfer is by far the fastest process, so that osmotic equilibration of two compartments is permanently ensured. Since the transports of the EC and the OR generally occur at different rates, the osmotic regulation process induces a continuous flow of water, varying the inner and outer volumes. Water transport may be critical for the various applications of multiple emulsions. For instance, swelling of the internal droplets may increase the effective globule fraction and produce significant changes in the rheological properties of the double emulsion.

Pioneer studies on double emulsions were performed in presence of small molecular weight surfactants. The intrinsic instability of such materials, namely fast coalescence and diffusion, precluded viable technological developments. Recent studies using amphiphilic polymers, proteins, and solid colloidal particles reveal that coalescence can be inhibited and that diffusive transport can be considerably slowed down.

There is still an interest in formulating double emulsions from sustainable and label-friendly ingredients and to reduce the number of additives to meet consumers' requirements. One of the main drawbacks encountered in food related applications is the lack of food-grade emulsifiers available to efficiently stabilize W/O emulsions. The most widely used lipophilic emulsifier is polyglycerol-polyricinoleate (PGPR). This emulsifier is highly effective for stabilizing W/O emulsions made with triglyceride oils. Nevertheless, its use is strictly regulated for many food products. Moreover, its presence is readily detected as an unpleasant off-taste when incorporated at the normally required level (i.e. 2-10 wt.%) for effective long-term stabilization of W/O emulsions. As a consequence, the use of PGPR represents one of the main technological drawbacks to the implementation of double emulsions in the food industry. Some recent efforts have been made to reduce the amount of PGPR in multiple emulsions, especially by incorporating hydrocolloids like casein, calcium alginate, acacia gum, or whey proteins in the inner droplets, that can interact with PGPR at the oil/water interface and increase the yield of encapsulated species. Frasch-Melnik, S., Spyropoulos, F., & Norton, I.T. (2010). (W1/O/W2 double emulsions stabilized by fat crystals--formulation, stability and salt release. Journal of Colloid and Interface Science, 350(1), 178-185, https://doi.org/10.1016/j.jcis.2010.06.039), proposed an alternative strategy to avoid the use of PGPR. W1/O emulsions based on sunflower oil and containing fat crystals were incorporated into an aqueous phase containing sodium caseinate to create kinetically stable W1/O/W2 double emulsions. The W1/O primary emulsion was stabilized by both monoglyceride, and triglyceride crystal "shells". The stability of the double emulsions under storage conditions was monitored over time. KCl encapsulated in the W1 phase of the primary emulsion was slowly released to the W2 continuous aqueous phase. Moreover, it was observed that the oil globules partially coalesced during storage. The stability of such composition thus needs to be further improved.

**Aims of the invention**

The present invention aims to solve the technical problem of providing a multiple, typically a double emulsion overcoming one or more of the above recited deficiencies.

The present invention aims to solve the technical problem of providing a multiple, typically a double emulsion comprising only food-grade emulsifiers.

In particular, the present invention aims to solve the technical problem of providing a multiple, typically a double emulsion without PGPR and more generally without any added lipophilic surfactant.

In particular, the present invention aims to solve the technical problem of providing a multiple, typically a double emulsion for use in a food, a cosmetic or a pharmaceutical composition.

The present invention aims also to solve the technical problem of providing a multiple, typically a double emulsion allowing the reduction of fat in a composition.

The present invention aims to solve the technical problem of providing a multiple, typically a double emulsion which does not negatively affect the organoleptic properties of a composition, and in particular providing a multiple, typically a double emulsion which does not negatively affect the taste of the composition containing such double emulsion.

The present invention aims to solve the technical problem of providing a stable multiple, typically double, emulsion, and more specifically sufficiently resisting to an osmotic mismatch between W1 and W2.

The present invention aims to solve the technical problem of providing a multiple, typically a double emulsion encapsulating one or more active ingredients and controlling the release thereof.

The present invention aims to solve the technical problem of providing a process for preparing such multiple, typically a double emulsion.

In particular, the present invention aims to solve the above recited technical problem in for the food industry, typically for preparing food compositions.

**Description of the invention**

The present invention provides a solution to the above recited technical problems.

More precisely, the present invention relates to a multiple emulsion comprising or consisting of P1/O/W2, wherein P1 is an aqueous phase forming droplets or a gas phase forming bubbles, said droplets or bubbles being dispersed in O thereby forming P1/O, wherein O is an oily phase comprising crystals, wherein W2 is an aqueous phase comprising at least one hydrophilic surfactant, wherein P1/O globules are formed in W2, wherein said oily phase O comprises at least 90%, preferably at least 92%, and even preferably at least 95%, by mass of triglycerides with respect to the mass of the O phase.

Such a multiple emulsion is obtainable by a process or method for preparing a multiple emulsion, said multiple emulsion being represented by P1/O/W2, wherein P1 is an aqueous phase forming droplets or a gas phase forming bubbles, said droplets or bubbles being dispersed in O thereby forming P1/O, wherein O is an oily phase comprising crystals, and W2 is an aqueous phase, and W2 is identical or different than P1, wherein said composition comprise less than 5% by mass of endogenous lipophilic surfactants with respect to the total mass of the multiple emulsion, wherein said process comprising the following steps:

- dispersing P1 in O, at a temperature where O contains crystals, to obtain a primary P1/O double phase; advantageously said crystals stabilize the primary P1/O double phase,
- dispersing ,P1/O in W2 at a temperature where O contains crystals, and wherein W2 contains one or more proteins to obtain a P1/O/W2 multiple emulsion.

It was discovered by the present inventors that such a process allows the preparation of a multiple emulsion having the required properties. Advantageously, the multiple emulsions of the present invention are kinetically stable.

Advantageously, a multiple emulsion according to the invention comprises less than 1% of lipophilic surfactants. This condition basically implies that the multiple emulsion according to the invention does not comprise any added lipophilic surfactant. Preferably, lipophilic surfactants are only present in the final multiple emulsion because they are naturally occurring in the raw materials, for example in natural oils, used to prepare the oily phase (O). Such endogenous lipophilic surfactants, like fatty acids, mono or diglycerides, are not *per se* efficient W/O emulsion stabilizers. The expression "multiple emulsion does not comprise any added lipophilic surfactant" means that no exogenous lipophilic surfactant has been incorporated in the formulation. Typically, a lipophilic surfactant is a surfactant having a hydrophile-lipophile balance (HLB) number of less than 10 and is therefore oil-soluble.

According to an embodiment, the oily phase O comprises at most, and preferably less than, 5% by mass of fatty acids, monoglycerides and diglycerides with respect to the mass of the O phase.

In one embodiment, said multiple emulsion does not comprise fat crystals made of saturated mono- or diglycerides.

The double emulsions according to Frasch-Melnik, S., Spyropoulos, F., & Norton, I. T. (2010) contained exogenous lipophilic surfactant. The release of KCl encapsulated in the W1 phase was attributed to the damage caused to the fat crystal shells during the secondary emulsification step used to obtain the double emulsion structure. After 6 weeks, partial coalescence of the globules was observed in such a prior art emulsion. Incorporation of a lipophilic surfactant in O/W emulsions based on crystallizable oils may induce partial coalescence. This instability is initiated in presence of fat crystals. Upon cooling, the spherical shape of the warm dispersed droplets which is controlled by surface tension evolves into a rough and rippled surface due to the formation of irregularly shaped/oriented crystals. When fat crystals are formed nearby the interface, they can protrude into the continuous phase and pierce the film between adjacent droplets, forming an irreversible link. This phenomenon is termed as partial coalescence since the shape relaxation process is frustrated by the intrinsic rigidity of the partially solidified droplets. The present invention overcomes this technical problem.

In one embodiment, multiple emulsions according to the invention comprise crystals of triglyceride-based edible fats.

In one embodiment, said multiple emulsion does not comprise lipophilic surfactant.

In the present invention, unless stated otherwise, droplets or gas bubbles represent the dispersed P1 phase in O (oil phase), and globules represent the dispersed P1/O emulsion in W2.

In one embodiment, air or gas bubbles (P1 phase) are dispersed in O and the globules represent the dispersed A/W foam in W2.

Advantageously, the multiple emulsion of the invention allows stabilizing the droplets and air (gas) bubbles within the oil phase, O.

In one embodiment, the multiple emulsion of the invention is a double emulsion.

**P1 phase**

In one embodiment, P1 is an aqueous phase forming droplets.

In one embodiment, P1 is a gas phase is air or a gas comprising nitrogen.

Advantageously, the incorporation of air in an oil phase is of significant interest for many applications such as cosmetics or personal care products. Oil foams can also be used in the food industry to reduce the fat intake without altering the sensory and textural properties. Concomitantly, the current trend is towards reducing the number of additives in many formulations. The invention relates in particular to multiple Air-in-Oil-in-Water (A/O/W) emulsions without any added lipophilic surfactant.

Preferably, the average diameter of P1 aqueous droplets ranges from 1 μm to 10 μm, preferably from 1 μm to 5 μm.

Preferably, the average diameter of P1 gas bubbles ranges from 5 μm to 20 μm.

Preferably, the average diameter of the fat globules ranges from 5 μm to 100 μm.

The average size of the droplets and globules is determined according to the method of example 2 by a Mastersizer 2000 Hydro SM.

The size of the gas bubbles is determined by direct imaging using an optical microscope equipped with a video camera. Images are recorded and the dimensions of about 350 droplets are measured, so that both the average diameter and the polydispersity index can be estimated:

$$D = D[4,3] = \frac{\sum N_i D_i^4}{\sum N_i D_i^3} \quad \text{and} \quad U = \frac{1}{\overline{D}} \frac{\sum N_i D_i^3 (\overline{D} - D_i)}{\sum N_i D_i^3} \quad (1)$$

where $N_i$ is the total number of droplets with diameter $D_i$. The median diameter, $\overline{D}$, is the diameter for which the cumulative undersized volume fraction is equal to 50%

Preferably, the polydispersity index, $U$, is less than 0.5, and preferably less than 0.4.

In one embodiment, P1 comprises a hydrophilic active ingredient, for example selected from the group consisting of hydrophilic pharmaceutically or cosmetic active ingredients, hydrophilic sensory active ingredients, and any combination thereof.

The hydrophilic sensory active ingredient is defined as a hydrophilic ingredient initially dissolved in P1, providing a benefit on a sensory point of view, and more particularly can be recognized by trained experts in the food, cosmetic or pharmaceutical industry with respect to their sensory benefit or lack of negative impact on a sensory property. For example, this ingredient should not detrimentally affect the taste and other sensory properties of a food product. Food products are known to be particularly sensitive to changes in taste, texture, color and other sensory properties. Advantageously, a sensory active ingredient provides a benefit to taste, texture, color and other sensory properties of a food, cosmetic or pharmaceutical composition.

In one embodiment, the hydrophilic sensory active ingredient is a food ingredient.

In one embodiment, the hydrophilic sensory active ingredient is a food aroma.

In one embodiment, the hydrophilic active ingredient is a compound providing a health and/or nutritional benefit.

A method for determining a benefit or absence of negative effect on a sensory point of view is the following: this method consists to understand the impact of product changes on consumer liking and acceptance qualitatively & quantitatively. From a qualitative perspective, the method aims to learn about perceptions & attitudes of small groups of people (up to 10 consumers) toward the hydrophilic active ingredient. From a quantitative perspective, either consumers (typically more than 100) or experts are asked to complete ballot questions and to rank sensory attributes that are specific to the products tasted in a monadic & sequential way, according to the liking, the overall appearance, the flavor and the texture.

After the fabrication of the double emulsion, the encapsulation yield of said active ingredient is of 20 to 80 % relative to the molar amount initially incorporated in P1.

**Oil phase (O)**

Preferably, O comprises or consists of one or more edible fats, for example selected from the group consisting of butter, milk fat, anhydrous milk fat, tallow, lard, mutton fat, poultry fat, fish oil, cocoa butter, palm oil, coconut oil, tree-nut oil, legume oil, sunflower oil, safflower oil, corn oil, cottonseed oil, soybean oil, canola oil, peanut oil, palm oil, palm olein, palm super-olein, palm kernel oil, algae oil, flaxseed oil, or any combination thereof.

Edible fats are preferably selected from the group consisting of triglycerides.

O may comprise one or more fatty acids and/or fatty esters, typically from vegetal or animal oils.

In one embodiment, O comprises or consists of one or more triglyceride-based edible oils (or fats) selected from the group consisting of natural vegetable and animal fats, optionally structurally rearranged or otherwise modified vegetable and animal fats, including any of combinations thereof.

For example, O comprises or consists of triglycerides with a melting range from -40 °C to +45 °C. Typically O comprises or is consisting of a mixture of triglycerides with a melting range from -40 °C to +45 °C.

O may comprise one or more oils (or fats) from various sources, for example it can be selected from the group consisting of butter, milk fat, anhydrous milk fat, tallow, lard, mutton fat, poultry fat, fish oil, cocoa butter, palm oil, coconut oil, sunflower oil, safflower oil, corn oil, cottonseed oil, soybean oil, canola oil, peanut oil, palm oil, palm olein, palm super-olein, palm kernel oil, algae oil, flaxseed oil, or any combinations thereof.

In one embodiment, the O is cholesterol-free or cholesterol-reduced.

Typically, O comprises or is consisting of short chain fatty acids (strictly less than 8 carbons), medium chains fatty acids and long chains fatty acids (strictly more than 14 carbons), optionally including saturated carbons. For example, the fatty acid chains contain from 12 to 22 carbon atoms, typically from 16 to 20 carbon atoms. In one embodiment, unsaturated chains may represent more than 20% of the total fatty acids. In one embodiment, O comprises oleic acid.

In one embodiment, phase O comprises or is consisting of milk fat, for example anhydrous milk fat.

Anhydrous milk fat is a complex mixture of triglycerides with a range of melting temperatures from -40 °C to +40 °C. Typically, it is composed of approximately 6 % short chain fatty acids (strictly less than 8 carbons), 20 % medium chains and 72 % long chains (strictly more than 14 carbons), including 42 % saturated C16 and C18 chains. In one embodiment unsaturated chains may represent from 25-30% (typically around 27 %) of the total fatty acids, oleic acid being the most abundant one with 20-25% (typically around 22 %).

Advantageously, the oil used according to the invention provides crystals stabilizing the P1/O/W emulsion.

In one embodiment, a tree-nut oil, a legume oil or any mixture thereof is used.

In one embodiment, oil may be transformed with the use of enzymes, in particular in an interesterification process or equivalent process such that the crystal content is sufficient to stabilize the P1/O/W emulsion. For example, such interesterification process or equivalent process may be implemented with a tree-nut oil.

Preferably, the viscosity of the primary P1/O emulsion remains low enough to process the emulsion under laminar flow conditions.

In one embodiment, the viscosity of the primary P1/O emulsion is ranging from 0.08 to 1 Pa.s, for example from 0.2 to 1 Pa.s according to the conditions as set forth in the viscosity test. Typically, the viscosity of P1/O is determined according to example 2, using an AR G2 controlled stress rheometer.

Advantageously, P1/O are stable under storage conditions owing to the presence of fat crystals.

In one embodiment, O comprises from 5 to 60%, preferably, less than 40 % by mass of P1 relative to the total mass of P1/O phases.

In one embodiment, said multiple emulsion comprises from 5 to 60%, preferably, from 10 to 40 % by mass of P1/O phases relative to the total mass of multiple emulsion.

In the present invention, reference is made to W1/O when P1 is an aqueous phase dispersed in the oily phase O and reference is made to A/O when P1 is a gaseous phase dispersed in the oily phase O (P1/O is a foam).

In one embodiment, W1 represents a mass fraction ranging from 10 to 60 % of the total dispersed phase P1/O.

In one embodiment, A represents a fraction ranging from 20 to 40%, the % being expressed in volume of gas with respect to the total A/O foam volume.

In an embodiment, A/O represents a non-aqueous foam.

Whereas aqueous foams have been extensively studied, research on non-aqueous foams is still at a pioneering stage. The prior art (such as Brun M.; Delample M.; Harte E.; Lecomte S.; Leal-Calderon F. Stabilization of Air Bubbles in Oil by Surfactant Crystals: A Route to Produce Air-in-Oil Foams and Air-in-Oil-in-Water Emulsions. Food Res. Int. 2015, 67, 366-375.) supports that foam stabilization is obtained by the addition of a compound that was not initially present in the oil phase. In the present invention, it was discovered that it is possible to obtain highly stable foams by whipping a crystallizable oil, for example anhydrous milk fat (AMF), without adding any external molecule (exogenous molecule) to the oil phase.

The invention relates to a new kind of product: Air-in-Oil-in Water (A/O/W) multiple emulsions, i.e. an oil-foam in an aqueous phase.

The application of W/O/W multiple emulsions is typically limited by their thermodynamic instability, especially their tendency for unwanted droplet-globule coalescence during the storage period. Another inconvenient of this type of W/O/W emulsion lies in the possible diffusion of water driven by any osmotic pressure difference between the aqueous compartments and of hydrophilic compounds from one aqueous phase to the other one through the oil layer due to the concentration mismatch.

Advantageously, in A/O/W emulsion of the invention, osmotic regulation is not an issue and coalescence of the inner bubbles on the globule surface can be ruled out since air and water are insoluble.

Advantageously, the A/O/W emulsion provides reduction of the fat intake.

Advantageously, the A/O/W emulsion provides novel applications like for example ultrasound imaging, notably owing to the large acoustical contrast provided by the air bubbles.

**W2**

In one embodiment, hydrophilic emulsifiers are selected from the group consisting of proteins, hydrocolloids, amphiphilic polymers, surfactants preferably having a HLB number higher than 10, i.e. having an affinity for water.

In one embodiment, W2 comprises at least one protein.

Proteins can be of animal or vegetal origin. These include for example caseins or whey proteins deriving from cow milk, and proteins deriving from any vegetal source like sunflower, rapeseed, soybean.

In one specific embodiment, the protein of W2 is sodium caseinate (NaCAS) (typically $M_w \approx 23,300$ g.mol$^{-1}$, typically containing 3 wt.% sodium).

Typically, W2 has a dynamic viscosity ranging from 0.1 to 1 Pa.s.

The viscosity of W2 can be measured using a conventional rheometer.

The viscosity of W2 should be preferentially higher than 0,1 Pa.s when the shear rate is 10,000 s-1.

In an embodiment, W2 is gelled. Advantageously, gelling avoids buoyancy driven phenomena of the globules (creaming).

In one specific embodiment, the invention relates to a A/O/W emulsions comprising a protein, for example sodium caseinate, as sole emulsifier.

In one specific embodiment, the invention relates to a A/O/W emulsions comprising anhydrous milk fat as O phase and a protein, for example sodium caseinate, as sole emulsifier in W2 phase.

Advantageously, a multiple emulsion according to the invention exhibits enhanced properties compared to conventional multiple emulsions including:

    i) very slow passive delivery of the encapsulated species,
    ii) osmotic stress resistance, and
    iii) thermo-responsiveness as the double globules could release the inner droplets' content upon warming.

Advantageously, a multiple emulsion according to the invention is stable for several months with no delivery of the encapsulated active species and no globule coalescence.

**Process**

The invention also relates to a method for preparing a multiple emulsion, typically a double emulsion.

In one embodiment, said process comprises:

1) Dispersing P1 in O, at a temperature where O contains crystals, to obtain a primary P1/O double phase; advantageously said crystals stabilize the primary P1/O double phase.

For example, such dispersing step comprises preparing an aqueous P1 solution comprising an active ingredient, and if needed the dissolution of the active ingredient in the aqueous solution. Techniques for dissolving a hydrophilic active ingredient in an aqueous solution, typically water, are known by a skilled person.

Preferably, prior to the dispersing step, the process comprises the full melting of O.

For example, the temperature T0 for melting O is above 25°C, typically above 30°C, above 40°C, above 50°C or above 60°C.

Preferably, after melting, O is cooled at a first temperature T1 to get fat crystals.

In one embodiment, after melting, O is cooled at T1 to get fat crystals at a first quantity, then O is heated to a second temperature T2 to get fat crystals at a second quantity.

Optionally, O is sheared to disaggregate crystals and to reduce its viscosity.

In one embodiment, the solid fat (crystals) content is ranging from 10 to 40%. The solid fat content can be measured using proton NMR and is expressed relative to the total proton content in O.

In one embodiment, the first solid fat content is ranging from 30 to 80%.

In one embodiment, the second solid fat content is ranging from 10 to 40%.

Preferably, prior to the dispersing step, the process comprises setting the aqueous solution P1 at the same or equivalent temperature as O. Then, preferably, P1 is incorporated in O at temperature T0 or equivalent.

In one embodiment, P1/O is then quenched by soaking in a cooling bath.

In one embodiment, the cooling rate is of P1/O is elevated in order to produce small fat crystals.

In one embodiment, the cooling rate is ranging from -1 to -10 °C/min.

In one embodiment, the cooling bath is ice water at 0°C.

In one embodiment, the cooling bath is a brine (salted water) at a temperature of less than 0°C, for example -5°C.

Typically, it is necessary to wait until P1/O reaches a temperature below the temperature of crystallization of fat crystals in O.

Preferably, after quenching, P1/O is sheared at a temperature below the onset crystallization temperature of fat crystals in O, for example at 20 °C.

To fabricate P1/O emulsion, an example of shearing conditions is the following: the system is sheared at a temperature below the onset crystallization temperature of O, using a mixer operating under turbulent flow conditions, for example with an Ultra-Turrax® T5, operating for example at 12 000 rpm, for a time sufficient to obtain a first P1/O emulsion, typically for 1-10 minutes, for example 2 min.

According to the invention, different mechanical treatments that can be implemented to fabricate W1/O emulsion including propellers, rotor stator devices, and high-pressure homogenizers.

To fabricate A/O foam, an example of shearing conditions is the following: O is whipped at a temperature below the onset crystallization temperature of O, using a propeller, for example a R1342 propeller stirrer, 4-bladed (IKA) rotated by an IKA RW20 motor, operating for example at 2 000 rpm, for a time sufficient to obtain a A/O foam, typically for 5-10 minutes, for example 5 min.

According to the invention, different mechanical treatments that can be implemented to fabricate A/O foam including propellers or any type of whipping device known by a skilled person.

2) dispersing P1/O in W2 to obtain P1/O/W2 emulsion

This second step requires a much finer tuning of the shear than the first one. Given the high viscosity of P1/O, a shear too low will not disperse the system correctly in W2. Conversely, a shear too intense will cause a release of the internal phase W1 or A.

For this reason, the applied shear must lie within in an optimal range. In one embodiment, the shear stress applied to P1/O in W2 is ranging from 1,000 to 10,000 s$^{-1}$.

When an emulsion is subjected to shear flow, the equilibrium shape of the droplets is governed by two dimensionless quantities: the dispersed-to-continuous viscosity ratio, $p = \eta_d/\eta_c$, and the capillary number defined as:

$$Ca = \frac{2\tau}{P_L} = \frac{D\eta_C\dot{\gamma}}{2\sigma} \quad (2)$$

Where $\eta_c$ is the viscosity of the continuous phase, $\dot{y}$ is the applied shear rate, $\sigma$ is the oil-aqueous phase interfacial tension, and $D$ is the droplet diameter (here the average diameter of the globules). The capillary number is the ratio of viscous stress to Laplace pressure, $P_L = 4\sigma/D$. The viscous stress tends to stretch the droplet out into a filamentary shape, whereas Laplace pressure (interfacial stress) tends to contract the droplet into a sphere. As the shear rate is increased, the capillary number also increases and the droplet becomes more elongated.

The average droplet diameter of the ruptured droplets is given by:

$$D = \frac{2C_{ac}\sigma}{\eta_c \dot{\gamma}} \quad (3)$$

Following Eq. (3), in order to fragment the droplets at relatively low shear rates, a large viscosity of the continuous phase is required.

In one preferred embodiment, the dispersed (P1/O)-to-continuous (W2) viscosity ratio $p = \eta_d/\eta_c$ is ranging from 0.1 to 5 and the shear rate $\dot{y}$ is ranging from 1,000s$^{-1}$ to 7,000s$^{-1}$.

According to the invention, the process comprises a first emulsification step to form an oleogel. In one embodiment, O is comprising or consisting of fat crystals dispersed in liquid oil.

In one embodiment, A/O forms a foam containing fat crystals.

Typically, increasing the applied shear rate results in a reduction of the size of the globules.

Preferably, the dispersion of P1/O in W2 is performed at a temperature where O contains crystals, by incorporating the primary W1/O emulsion or the A/O foam into the external aqueous phase, W2, under stirring.

Preferably, the osmotic pressure in W2 is the same as in W1 phase. Advantageously, this avoids water transfer phenomena.

In one embodiment an osmotic modulating agent, for example, glucose may be added to W2, notably to match the osmotic pressure.

Preferably, the second emulsification step (P1/O in W2) is performed less than 30 minutes after preparing of P1/O.

Preferably, the process comprises shearing P1/O in a viscous aqueous phase W2 under laminar flow conditions.

The shear must be applied at a temperature where O contains crystals.

Such a shear may be produced, for example, in a cell consisting of two concentric cylinders, at least one cylinder being in rotation relative to the other, such as a Couette cell (TSR, France; concentric cylinders' configuration). In one embodiment, the inner cylinder is rotating and the outer cylinder is immobile. In this type of cell, the shear is then defined by the number of rotations per minute and the gap between the two cylinders (for example from 100 to 300$\mu$m, typically 200$\mu$m).

Advantageously, the shearing conditions allow controlling the shear, making the droplets of dispersed phase monodisperse and controlling the size of the droplets and/or globules.

For example, the shear stress applied to P1/O/W2 is ranging from 1,000 to 10,000 s$^{-1}$.

A Couette cell with a thin gap (typically 100 $\mu$m) ensures a spatially homogeneous shear. According to the invention, double emulsions can also be obtained using mixers that do not apply a spatially homogeneous shear over the whole emulsified volume like rotor-stator devices and propellers.

According to the invention, the process comprises a step of dissolving one or more proteins in W2 prior to the second emulsification step.

In one embodiment, P1 is mixed with an oil phase (O) without adding surfactant at a temperature above O melting range; P1/O system is cooled down to a temperature below the onset crystallization temperature of O to make fat crystallize under stirring, then P1/O emulsion is dispersed in a highly viscous external aqueous phase W2 containing a protein to produce a P1/O/W2 double emulsion.

Advantageously, said P1/O/W2 multiple emulsion is stored under conditions wherein O contains crystals.

Such storage is for example a storage at a temperature such that O contains crystals, for example below 30°C.

Advantageously, the dynamic viscosity of P1/O system is low enough to process the material under laminar flow conditions.

Advantageously, the shear rate for dispersing P1/O in W2 is ranging from 1,000 to10,000 s$^{-1}$.

## Method for preparing composition

The invention also relates to a method for preparing composition, preferably a food, a cosmetic or a pharmaceutical composition, said method comprising incorporating a multiple emulsion as defined in the present invention or as obtainable by a method as defined in the present invention into a base composition to form said composition.

In one embodiment, said composition is a food composition.

In one embodiment, said composition is a fresh dairy product.

In one embodiment, said composition is a fresh cultured dairy product.

In one embodiment, said composition is a yogurt.

In one embodiment, said composition is an ice-cream.

In one embodiment, said composition is a cultured plant-based product.

Preferably said base composition is a reduced fat base composition.

The term "base composition" means a formulation that comprises ingredients to which the multiple emulsion of the invention can be added to form a (final) composition.

In one embodiment, the multiple emulsion of the invention forms a mix, in particular a protein mix, for the preparation of yogurts or ice-creams.

In one embodiment, W1, O and W2 are pasteurized.

In one embodiment, the multiple emulsion of the invention is fermented.

**Composition**

The invention relates to a composition, preferably a food, a cosmetic or a pharmaceutical composition comprising a multiple emulsion as defined in the present invention or as obtainable by a method as defined in the present invention.

In one embodiment, said composition is a food composition.

In one embodiment, said composition is a fresh dairy product.

In one embodiment, said composition is a fresh cultured dairy product.

In one embodiment, said composition is a yogurt.

In one embodiment, said composition is an ice-cream.

In one embodiment, said composition is a cultured plant-based product.

Preferably said composition is a reduced fat composition.

The present invention also relates to packaging containing a composition according to the invention.

Such packaging could be for example in the form of one-portion multiple packaging or larger-portion packaging.

In one embodiment, the package is a yogurt or ice-cream container.

**In the figures:**

Figure 1: Evolution of the temperature in a W1/O emulsion containing 30 wt.% of aqueous droplets when quenched in (a) pure water (0 °C) and (b) a brine solution (-5 °C). Temperature was registered using a thermocouple plunged in the emulsions.

Figure 2: Microscopic images of a primary W1/O emulsion containing 30 wt.% of a 0.5 mol.L-1 NaCl aqueous phase dispersed in Anhydrous Milk Fat (AMF), after being processed in a cooling bath (a) at T = -5°C, and (b) at T = 0 °C.

Figure 3: Evolution of the size distribution with the applied shear rate for double emulsions initially composed of 30 wt.% of W1/O globules containing 30 wt.% of W1 droplets, stabilized by 12 wt.% NaCAS in the external aqueous phase. Figure 3 includes microscope images of the emulsions obtained at the two limiting shear rates (1,050 s$^{-1}$ (b) and 7,350 s$^{-1}$(a)). Emulsions were diluted with an isotonic D-glucose solution to facilitate the observation.

Figure 4: Evolution of the volume-averaged diameter with the applied shear rate for double emulsions initially composed of 30 wt.% of W1/O globules containing 30 wt.% of W1 droplets stabilized by 12 wt.% NaCAS in the external aqueous phase. Dots are mean values $\pm$ SD of measurements from 3 different samples.

Figure 5: Flow curves of the external aqueous phase, $W_2$, and of the $W_1$/O emulsions at different inner droplet fractions.

Figure 6: Evolution of the average globule diameter with the initial inner droplet fraction for multiple emulsions initially composed of 30 wt.% of $W_1$/O globules dispersed in an aqueous phase containing 12 wt.% NaCAS. The applied shear rate was equal to 5 250 s$^{-1}$. Dots are mean values $\pm$ SD of measurements from 3 different samples.

Figure 7: Evolution of the encapsulation yield as a function of the applied shear rate, for multiple emulsions initially composed of 30 wt.% of $W_1$/O globules containing 30 wt.% of $W_1$ droplets, dispersed in a 12 wt.% NaCAS aqueous phase. Dots are mean values $\pm$ SD of measurements from 3 different samples.

Figure 8: Evolution of final inner droplet fraction as a function of the initial one for multiple emulsions initially composed of 30 wt.% of $W_1$/O globules dispersed in a 12 wt.% NaCAS aqueous phase. The applied shear rate was equal to 5,250 s$^{-1}$. The dotted line is a guide for the eyes.

Figure 9: Measurement of the encapsulation yield during storage at 4 °C for a multiple emulsion initially composed of 30 wt.% of $W_1$/O globules containing 30 wt.% of $W_1$ droplets, sheared at 5,250 s$^{-1}$.

Figure 10: Stability study of a multiple emulsion initially composed of 30 wt.% of $W_1$/O globules containing 30 wt.% of $W_1$ droplets, dispersed in 12 wt.% NaCAS in the external aqueous phase: (a) without dilution; (b) 3-fold dilution in an aqueous phase containing 0.8 mol.L$^{-1}$ D-Glucose. The images were obtained after a storage period of 21 days.

Figure 11: Emulsions 1 and 2 submitted to a 10-fold dilution with (a) pure water and (b) an iso-osmotic solution. Observations were performed after 2h-storage.

Figure 12: multiple emulsions initially composed of 30 wt.% of $W_1$/O globules containing 30 wt.% of $W_1$ droplets, dispersed in 12 wt.% NaCAS aqueous phase. The oil phase used is (a) cocoa butter; (b) palm oil; (c) coconut oil.

Emulsions were diluted with an isotonic D-glucose solution to facilitate the observation.

Figure 13: Variation of the overrun as a function of temperature after a whipping period of 5 min. The dotted line is a guide for the eyes.

Figure 14: Variation of the overrun as a function of time at 20 °C. The dotted line is a guide for the eyes.

Figure 15: micrographs of the A/O foams before and after the refining step in the Couette cell.

Figure 16: Size histogram of the air bubbles

Figure 17: micrograph of the A/O/W emulsion sheared at 3,150 s-1 and corresponding size distribution.

Figure 18: Influence of the shear rate on the volume-averaged diameter of the fat globules of a A/O/W multiple emulsion. Dots are mean values of at least 4 sample measurements.

Figure 19: Microscopic images of the multiple A/O/W emulsion.

Figure 20: Quantification of encapsulation yield of air in the fat droplets for multiple emulsions obtained at different shear rates in a Couette cell.

Figure 21: Partial Coalescence of the multiple droplets after 1 day of storage at 4 °C.

Figure 22: Micrographs of the A/W/O double emulsion obtained at 3,150s-1 in the Couette cell. (a) t=0; (b) t=15 days, (c) t=1 month.

Figure 23: W1/O emulsion size distribution & microscopic observations of the double emulsion structure in yogurts.

**Examples**

**Example 1** - **Preparing a double emulsion according to the invention (W1/O/W2)**

According to the examples, the fabrication of double emulsions according to the invention involved the two following steps.

**- First step, $W_1$/O emulsion**

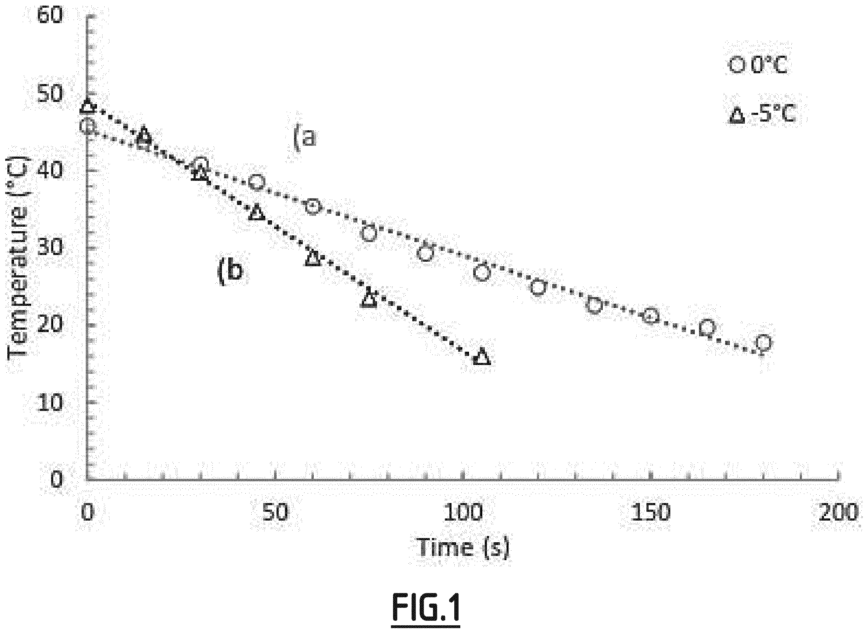

The $W_1$ aqueous phase was prepared by dissolving NaCl at 0.5 mol.L$^{-1}$. This solute was used as a tracer to measure the encapsulation yield, the release kinetics and also as a stabilizing agent to avoid coarsening phenomena. Anhydrous Milk Fat (AMF) was totally melted at T = 65 °C and the W1 phase, warmed at the same temperature, was progressively incorporated in the oil phase under manual stirring up to a fraction ranging from 10 to 60 wt.%. In total, 50 g were processed in a 100-mL beaker. The system was then quenched by soaking the beaker in a large-volume cooling bath (500 mL). Two different quenching conditions were experienced, corresponding to two compositions of the cooling bath: one based on ice water at 0 °C and the other based on a 10 wt.% NaCl brine solution at -5 °C. Figure 1 shows the evolution of the temperature in the emulsion containing 30 wt.% of aqueous phase for the two different cooling baths. Once the emulsion temperature of 20 °C reached, the system was sheared using an Ultra-Turrax® T5 mixer operating at 12,000 rpm for 2 min to obtain the final primary water-in-oil ($W_1$/O) emulsion.

**- Second step, $W_1$/O/$W_2$ emulsion.**

Right after the first step, a coarse multiple $W_1$/O/$W_2$ emulsion was prepared by incorporating 30 g of the primary $W_1$/O emulsion into 70 g of the external aqueous phase, $W_2$, under manual stirring, at 20 °C. The external aqueous phase was composed of 12 wt.% NaCAS, 0.8 mol.L$^{-1}$ D-glucose to match the osmotic pressure of the inner aqueous phase, $W_1$, and consequently to avoid water transfer phenomena. The concentration of glucose was selected using tabulated values from the Handbook of Chemistry and Physics (Handbook of Chemistry and Physics 98th Edition, 2017). The osmotic contribution of NaCAS was neglected due to its high molecular mass and the reduced amount of sodium ions (3 wt.%).

Quasi-monodisperse multiple emulsions were finally obtained by shearing the pre-emulsions within a narrow gap in a Couette cell (TSR, France; concentric cylinders' configuration) at 20 °C. The inner cylinder of radius r = 20 mm is moved by a motor that rotates at a selected angular velocity, $\omega$, which can reach up to 78.5 rad.s$^{-1}$. The outer cylinder is immobile, and the gap between the stator and the rotor is fixed at e = 200 $\mu$m. For the maximum angular velocity, we are able to reach very high shear rates, $\dot{\gamma}$ = r($\omega$/e = 7,850 s$^{-1}$, in simple shear flow conditions. The average multiple droplet size was finely tuned by varying the shear rate. The final emulsions were stored at 4 °C for several weeks.

**Example 2 - Emulsion characterization**

**2.1. Droplets characterization**

Both the primary $W_1$/O and the double $W_1$/O/$W_2$ emulsions were observed using an optical microscope Olympus BX51 (Olympus, Germany) equipped with an oil immersion objective (X 100/1.3, Olympus, Germany) and a digital color camera (Olympus U-CMAD3, Germany). When necessary, a cross-polarizing configuration of the microscope was adopted to visualize AMF crystals.

The droplet size distribution of the primary $W_1/O$ emulsion was measured using a Mastersizer 2000 Hydro SM from Malvern Instruments S.A (Malvern, UK). Measurements were performed directly after emulsification. Static light scattering data were transformed into size distribution using Mie theory (Mie, 1908), by adopting a refractive index of 1.47 for sunflower oil and of 1.34 for the $W_1$ aqueous phase. The sample (1 mL) was first diluted in sunflower oil (10 mL) containing PGPR (Polyricinoleate of polyglycerol) at 4 wt.% at room temperature. A small volume of the diluted $W_1/O$ emulsion was then introduced under stirring in the dispersion unit containing sunflower oil. Because of the very high dilution factor, AMF crystals were fully dissolved and the light scattering signal was only due to $W_1$ droplets stabilized by PGPR.

To measure the average size of the oil globules, 1 mL of $W_1/O/W_2$ emulsion was diluted in 10 mL of a SDS (Sodium Dodecyl Sulfate) solution at $8 \times 10^{-3}$ mol.L$^{-1}$. This surfactant has the ability to dissociate protein aggregates that bridge emulsion drops. A small volume of sample was then introduced under stirring in the dispersion unit containing a solution of Tween® 80 at $1.2 \times 10^{-5}$ mol.L$^{-1}$ to avoid foaming and droplet deposition on the optics, and 0.8 mol.L$^{-1}$ D-glucose to match the osmotic pressure of $W_1$ droplets within the globules. In this case, since the globules were not optically homogeneous due to the presence of the inner water droplets and fat crystals, the size distributions were obtained using Fraunhofer's model.

The size distributions of the emulsions were analyzed right after preparation and after a one-week storage at 4 °C. The emulsions were characterized in terms of their volume-averaged diameter D [4;3] and polydispersity index, U, defined in Eq. (1).

## 2.2. Viscosity measurements

The viscosity of $W_1/O$ emulsions and of the external aqueous phase $W_2$ were measured separately using an AR G2 controlled stress rheometer (TA Instruments, Delaware, USA). A cone-plate geometry of 60 mm diameter was adopted, with a cone angle equal to 2°, and a gap of 56 $\mu$m. Samples were submitted to a ramped increase in shear stress ranging from 100 to 5,000 s$^{-1}$ at 20 °C.

## 2.3. Differential Scanning Calorimetry (DSC) experiments

Thermal analyses were conducted on a differential scanning calorimeter (Setaram, micro DSC VII), using hermetically sealed aluminum pans (0.7 mL) as sample containers. DSC measurements were performed on a $W_1/O$ emulsion composed of AMF and 30 wt.% of dispersed aqueous droplets. The emulsion was stored at 20 °C and measurements were performed at different times after its preparation: t = 0 min; t = 45 min and t = 24 h. Samples were first kept at 20 °C for 5 min and then warmed up to 50 °C at + 2 °C.min$^{-1}$.

## 2.4. Conductivity measurements

The ability of multiple emulsions to retain the solute (NaCl) encapsulated in the internal aqueous phase ($W_1$) during the second emulsification step and under storage conditions, was evaluated by measuring the amount of solute released in the external phase ($W_2$). For that purpose, conductivity measurements were performed at room temperature using a Consort C931 conductimeter (Consort bvba, Belgium). A calibration curve was first obtained from solutions containing 6 wt.% NaCAS, and NaCl at different concentrations.

A small amount of the double-emulsions was collected and submitted to a 2-fold dilution using double-distilled water. The samples were then centrifuged at 1,500 g (g being the earth gravity constant) for 5 min with a Rotanta 460 RF centrifuge (Hettich Lab technology, Germany) in order to separate the globules from the external aqueous phase. Conductivity measurements were performed on the subnatant phase devoid of globules, and salt concentration was deduced from the calibration curve. It was checked that the applied centrifugation did not lead to coalescence phenomena that could produce further release of salt. For that purpose, the cream obtained by centrifugation was redispersed and observed under the microscope. Both the internal droplet size and the droplet concentration within the globules remained apparently invariant.

Primary $W_1/O$ emulsions containing 30 wt.% of internal aqueous droplets were first prepared without any added lipophilic surfactant in the oil phase. The emulsions were obtained by heating AMF at 60 °C, above its melting range, incorporating the aqueous phase and cooling the mixture to 20°C under intense stirring to form an oleogel. The equilibrium solid fat content of AMF at 20 °C is around 20%. At this temperature, the viscosity of the AMF oleogel was high enough to facilitate emulsification but low enough for easy handling. Two quenching rates were implemented (see Fig. 1).

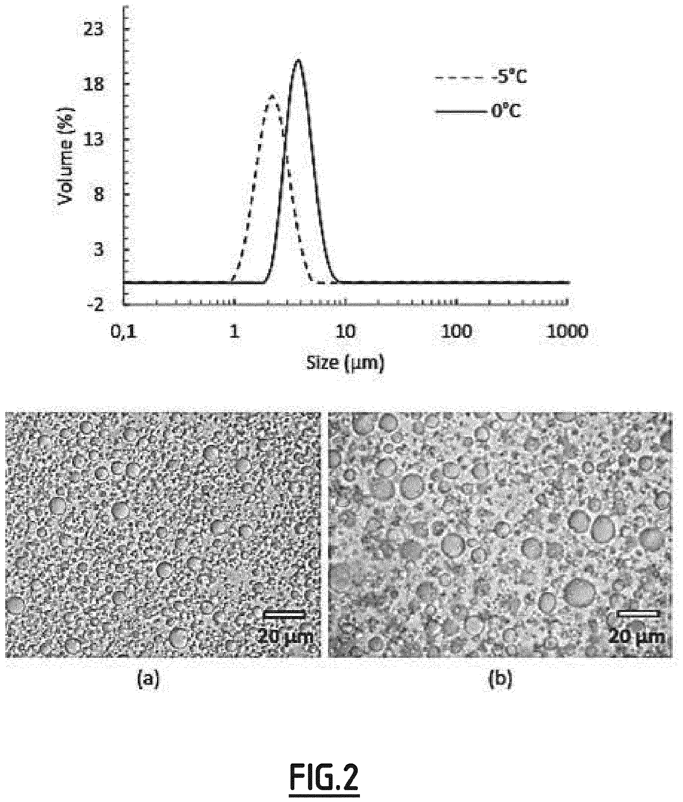

Fig. 2 shows microscope images of the obtained emulsions and the corresponding size distributions of the water droplets. The emulsion resulting from the slowest quenching conditions (using ice water at 0 °C) had an average diameter, D= 3.74 $\mu$m, larger than that resulting for the fastest quench (using a cooling bath at -5 °C), D= 2.15 $\mu$m. Both emulsions had relatively narrow size distributions with $U$ = 0.22 and $U$ = 0.26, respectively. The difference in the droplet average diameter emphasizes the impact of the cooling rate on the emulsification process. Hereafter, $W_1$/O emulsions will be formed using the fastest quench obtained with the brine solution at -5 °C. The emulsions were kinetically stable when stored at 20 °C or at 4 °C, as reveled by the size distributions that did not vary after several weeks of storage. Instead, macroscopic separation into the two immiscible phases took place within a few hours when the primary $W_1$/O emulsions were warmed at 50 °C, a temperature such that the oil phase is fully molten. This observation reveals that fat crystal ensure stabilization of the emulsions.

Fat crystals can be present in the continuous phase or at the interface. The stabilization of oil-continuous emulsions with fat crystals is thus often described as a combination of Pickering stabilization due to surface-active crystals adsorbed at the interface, and network stabilization due to physical trapping of droplets in the crystal network.

Crystal size and shape are mainly controlled by the cooling rate and by the flowing conditions.

## Example 3 - Multiple $W_1$/O/$W_2$ emulsions

### 3.1 Influence of shear rate

The obtained $W_1$/O emulsions were viscous pastes prone to harden over time. The freshly obtained emulsions were relatively fluid but, after a few hours at 20 °C, they exhibited considerable yield stress and firmness.

Crystallization of milk fat is a slow and complex process involving variable polymorphic forms. If the final temperature of the quench exceeds 13 °C, metastable b' crystals first appear at short times. Then, they progressively evolve toward the stable $\beta$ form. $W_1$/O emulsions of the invention were observed under polarized light microscopy immediately after their fabrication. Polarized light microscopy revealed small bright spots reflecting the formation of tiny crystals, probably of the $\beta$' form. Some Maltese crosses are also discernable. They are generally observed in spherulite-like crystals or in systems undergoing interfacial crystallization.

DSC measurements were performed on a $W_1$/O emulsion containing 30 wt.% of internal aqueous droplets after different storage periods at 20 °C: t = 0; t = 45min; t = 24h. The shape of the thermograms evolves over time, as a result of the polymorphic transition taking place.

The second emulsification step was always carried out less than 30 min after the fabrication of the $W_1$/O emulsion to avoid any significant polymorphic and structural evolution of the fat crystals. In this way, the viscosity of the primary emulsion remained low enough to process the material under laminar flow conditions. Some preliminary trials, were carried out by submitting the mixture of the two phases ($W_1$/O emulsion and $W_2$ aqueous phase) to vigorous agitation by means of an Ultra-Turrax mixer, operating at 12 000 rpm for 2 min. Multiple emulsions were obtained but the turbulent mixing resulted in a significant release of the inner droplets. The obtained emulsions were polydisperse and a significant fraction of the globules did not contain inner aqueous droplets. From these preliminary experiments, it was concluded that mild conditions in terms of agitation were required during the second emulsification step to preserve the multiple structure. An alternative strategy was thus adopted based on the application of a shear under laminar flow conditions in a highly viscous aqueous phase.

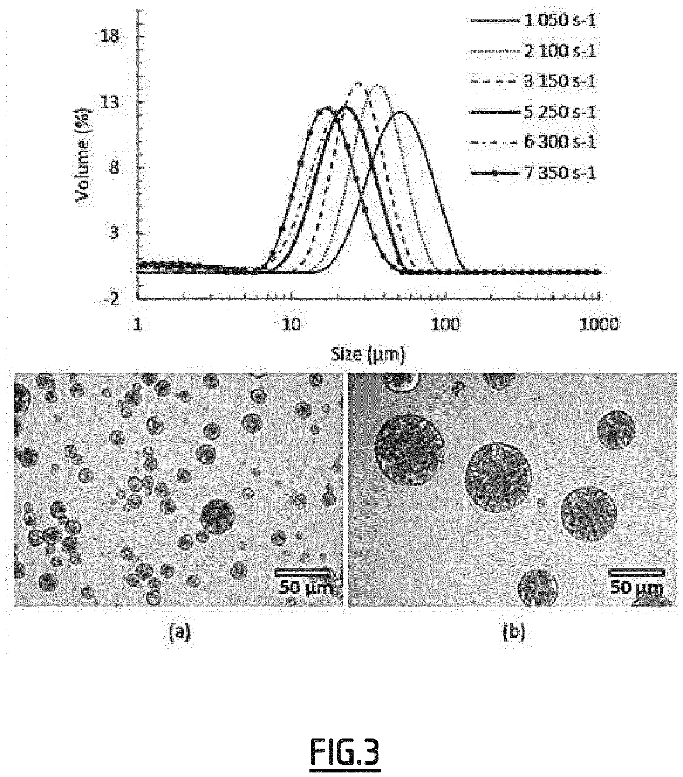

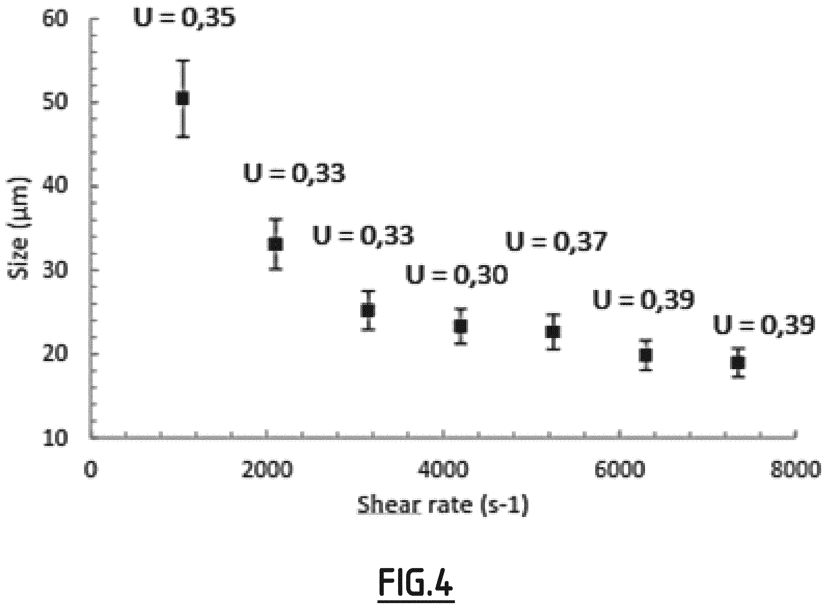

Multiple globules were obtained following the second step described in example 1. The applied shear in the Couette's cell was varied from 1,050 to 7,350 s$^{-1}$. Fig. 3 shows the evolution of the size distributions of the multiple globules obtained from a $W_1$/O emulsion initially containing 30 wt.% of aqueous droplets: as expected, increasing the applied shear rate resulted in a reduction of the size of the globules. All emulsions exhibited narrow size distributions, as reflected by the relatively low value of the polydispersity index, $U$ (<0.4). In Fig. 3 (a/b), we report microscope images of the emulsions obtained at the two limiting shear rates (1,050 s$^{-1}$ (a) and 7,350 s$^{-1}$ (b)). Large oil globules uniform in size are visible, and the smaller $W_1$ water droplets are also distinguishable. It can be concluded that the applied fragmentation method successfully produces compartmented globules with a relatively narrow size distribution, whose average diameter can be tuned by means of the applied shear rate. Fig. 4 shows the evolution of the average globule diameter, $D$, as a function of the shear rate, $\dot{\gamma}$. The polydispersity index, $U$, is indicated nearby each experimental point.

The viscosities of the $W_1$/O emulsion and of the $W_2$ continuous phase were measured by submitting each phase to a ramp in shear rate from 100 to 5,000 s$^{-1}$, at 20 °C. It turned out that measurements were not reproducible for emulsions whose internal water fraction, $\phi_i^0$, was larger than 30 wt.%. Wall slipping occurred due to coalescence phenomena of the aqueous droplets on the solid surfaces of the rheometer, ultimately leading to the formation of an aqueous layer. This phenomenon was not observed for emulsions with lower droplet fractions, allowing a more reliable determination of their viscosities. Also, it must be emphasized that the shear rates applied in the Couette cell, was as high as 7,350 s$^{-1}$, beyond the accessible range of our rheometer (<5,000 s$^{-1}$).

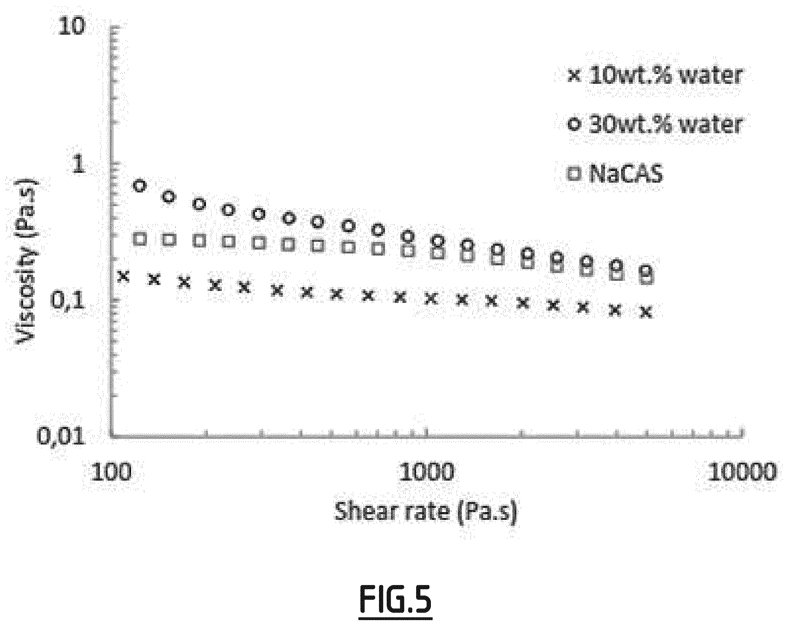

Fig. 5 shows the flow curves of the $W_1/O$ emulsions at various droplet fractions (10 and 30 wt.%) and of the external aqueous phase, $W_2$. All systems exhibit shear-thinning behavior.

On Fig. 5, it can be seen that the viscosities of the $W_1/O$ emulsions and of the continuous phase are close to each other at the highest attainable shear rate of 5,000 s$^{-1}$. This suggests that the viscosity ratio, $p$, is close to unity under the conditions of the emulsification process.

In the present study, the same condition holds, as both the inner droplets and fat crystals are sufficiently small compared to the globule size. From Eq. (2), it is possible to derive an estimated value of the critical capillary number. By adopting the following values: D= 22.5 $\mu$m, $\dot{\gamma}$ = 5,250 s$^{-1}$, $\sigma$= 4 mN.m$^{-1}$ (measurement performed using the rising drop method ("Tracker" apparatus from Teclis Instruments (France) at 45 °C), $\eta_c$= 0.2 Pa.s (value extrapolated from Fig. 5), Cac≈3 can be obtained.

### 3.2 Influence of inner droplet fraction

The internal water fraction was varied from 20 to 60 wt.%. At large droplets fractions $(\phi_i^0 > 50 \text{ wt.\%})$, $W_1/O$ emulsions were prone to phase inversion. To prevent this instability, the $W_1$ phase was always added progressively in AMF, within a time scale of 1 min. The average droplet size was close to 2-3 $\mu$m in all cases, but the polydispersity tended to increase with $\phi_i^0$ : $U$= 0.21 for $\phi_i^0 = 10 \text{ wt.\%}$ and $U$= 0.57 for $\phi_i^0 = 50 \text{ wt.\%}$. As explained, the $W_1/O$ emulsion was obtained using a turbulent mixer. Under these conditions, the final size distribution results from both droplet breakup and recombination (coalescence). The observed widening of the size distribution at large droplet fractions is probably due shear-induced coalescence during the emulsification process, which tends to become increasingly pronounced as the droplet fraction increases.

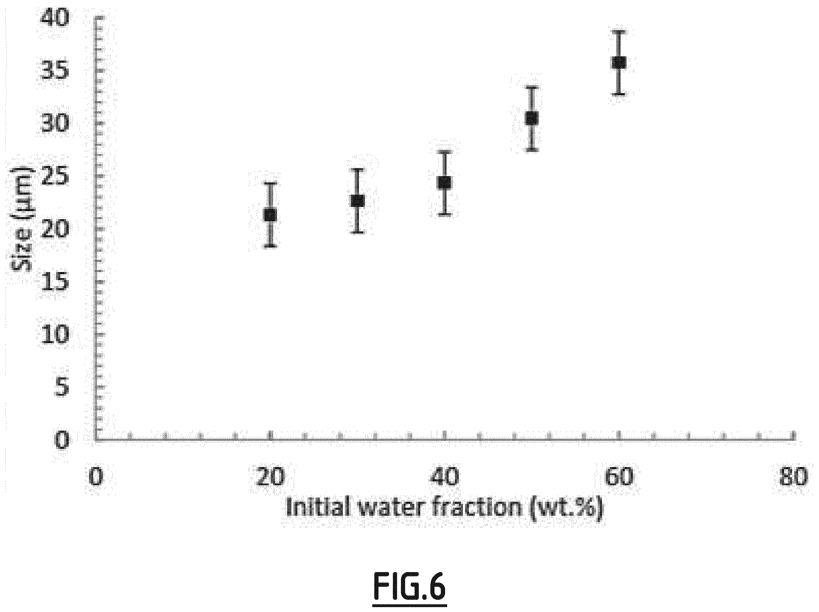

Once fabricated, the primary $W_1/O$ emulsion was in turn emulsified at 30 wt.% in the external aqueous phase, $W_2$. The shear rate in the Couette's cell was fixed at 5,250 s$^{-1}$. In Fig. 6, the evolution of the average globule size is plotted as a function of $\phi_i^0$. It can be observed that the globule size increases with the droplet mass fraction. This trend can be rationalized considering that the viscosity of the $W_1/O$ emulsion increases with the droplet fraction, as evidenced in Fig. 5. These results support the fabrication of quasi-monodisperse $W_1/O/W_2$ emulsions based on liquid oils.

### 3.3. Encapsulation yield

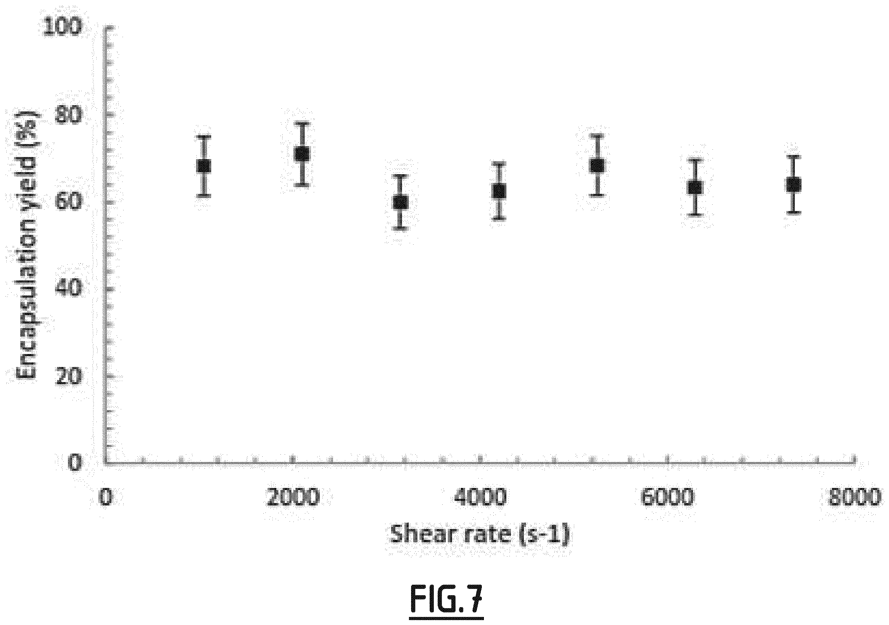

To evaluate the encapsulation yield, a quantification of the salt released in the external $W_2$ phase was performed directly after emulsification in the Couette's cell at variable shear rates from 1,050 to 7,350 s$^{-1}$. For that purpose, conductivity measurements were carried out on the external aqueous phase of each emulsion. The emulsions comprised 30 wt.% of globules, with $\phi_i^0 = 30 \text{ wt.\%}$. In Fig. 7, it can be seen that the encapsulation yield is only weakly varying. Over the explored range of shear rates, the average value of the final internal aqueous droplet fraction is 22 wt.%, corresponding to an average encapsulation yield of 72 %.

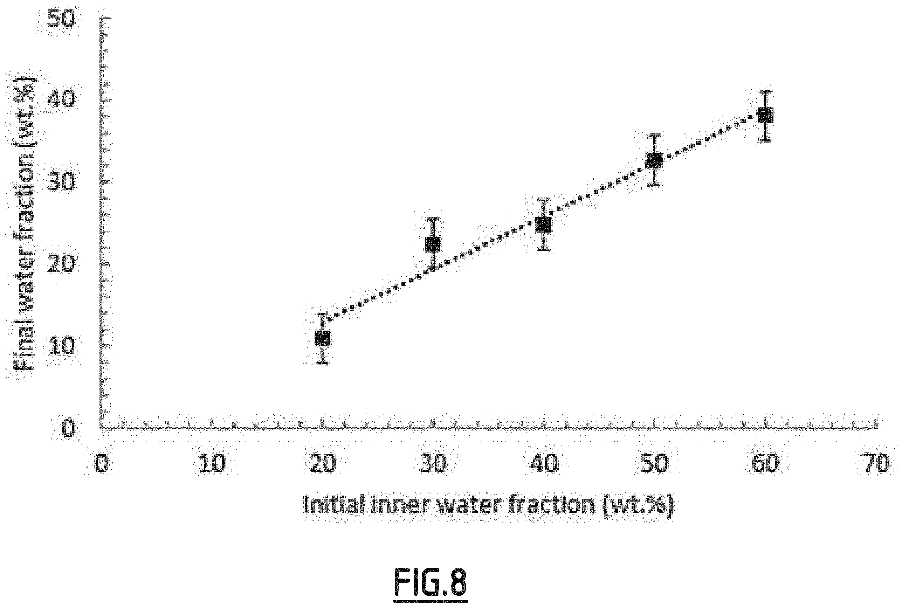

The evolution of the final internal aqueous fraction was also measured as a function of the initial one, at constant shear rate of 5,250 s$^{-1}$. The initial globule fraction was always equal to 30 wt.%. Fig. 8 reveals that the final water fraction is roughly proportional to the initial one. The linearity enables a fine tuning of the final inner droplet content, as required in many practical applications, with an average encapsulation yield close to 70 %.

### 3.4. Stability assessment

#### 3.4.1. Thermal responsiveness

The obtained double emulsions were thermally-sensitive. They could turn from $W_1/O/W_2$ emulsions to simple W/O ones upon warming, within a short period of time. When heated above 45 °C, the oil phase was fully molten and this resulted in the fast release of the inner droplet, as revealed by conductivity measurements, using NaCl as a delivery probe. Full release was achieved after less than 10 min.

### 3.4.2. Encapsulation ability

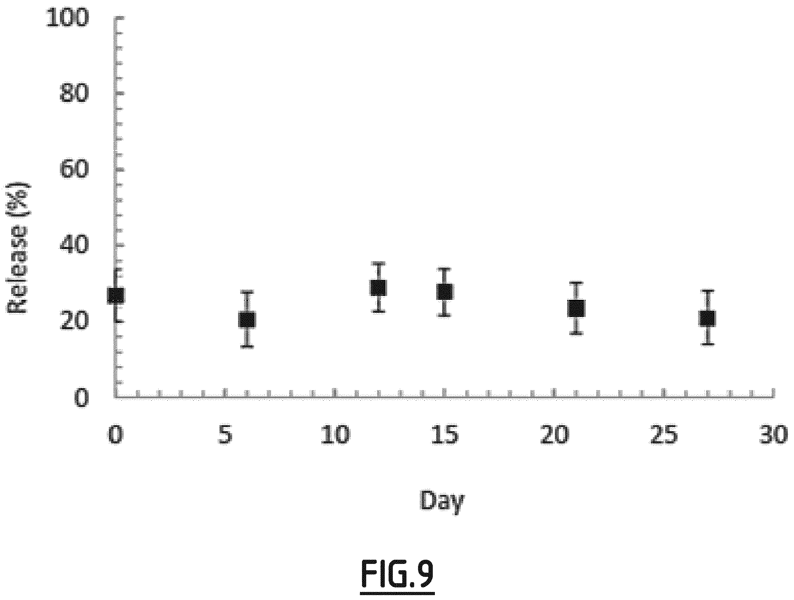

An emulsion with 30 wt.% of globules, $\phi_i^0 = 30$ wt.%, sheared at 5,250 s$^{-1}$ was stored at 4 °C for almost 1 month in order to measure the percentage of salt released from the inner droplets to the external aqueous phase under quiescent storage conditions. The results are reported on Fig. 9. The initial value (t=0) corresponds to the delivery provoked by the emulsification process. Surprisingly, the delivery did not evolve over time, reflecting an outstanding encapsulation ability of this type of double emulsion.

### 3.4.3. Resistance to partial coalescence

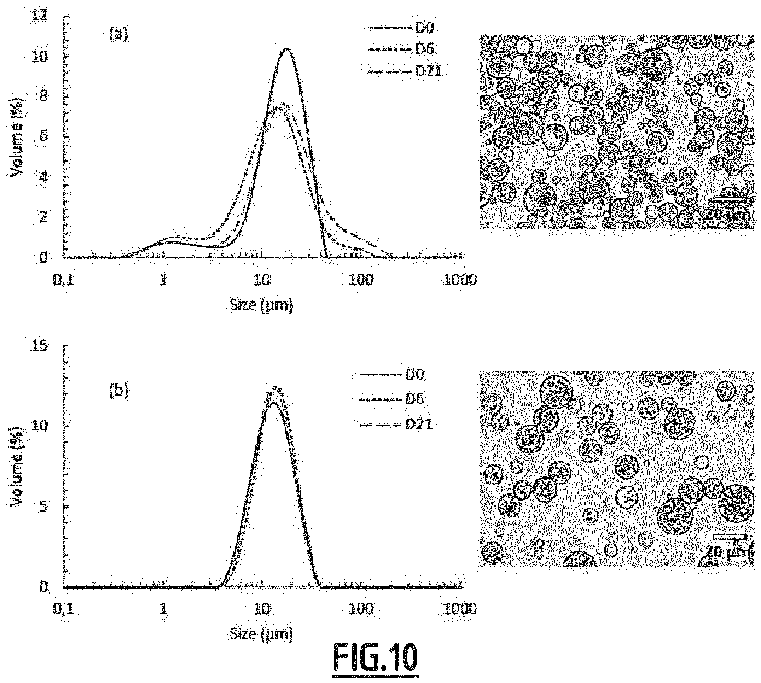

In order to probe its resilience to compositional changes and to partial coalescence, the emulsion was submitted to a 3-fold dilution under iso-osmotic conditions, using a 0.8 mol.L$^{-1}$ D-glucose aqueous solution. The targeted composition was the following: 10 wt.% globules; $\phi_i^0 = 30$ wt.%; 0.5 mol.L$^{-1}$ NaCl in $W_1$; 4 wt.% NaCAS, and 0.8 mol.L$^{-1}$ D-glucose in $W_2$. The stability at 4 °C was followed during 21 days by droplet sizing measurements and microscopic observations. The non-diluted emulsion was considered as a reference system (Fig. 10a). First, the emulsion was diluted with a solution containing D-glucose alone. Because of the low viscosity of the continuous phase (W2), the globules tended to form a dense cream after a few hours of settling. However, the cream was readily redispersable upon manual shaking, with no apparent sign of coalescence. Fig. 10b reveals that both the average globule size and the inner droplet fraction remained almost constant over the explored time interval. To avoid globule creaming, carrageenan was introduced in W2 at a concentration of 0.5 wt.% and again neither the size distribution nor the apparent internal structure of the droplet underwent any significant change.

### 3.5. Resistance to an osmotic shock - Swelling process

The resistance to osmotic stress of two double emulsions was compared, one based on AMF devoid of lipophilic surfactant (emulsion 1), and the other one based on a liquid oil (sunflower) and PGPR (emulsion 2).

Both systems were fabricated following the protocol described in the emulsion preparation described above in example 1. For emulsion 2, during the first step, a 1:1 (wt./wt.) mixture of the $W_1$ aqueous phase (0.5 mol.L$^{-1}$ NaCl) and of oil phase (sunflower oil + 3 wt.% PGPR) was submitted to an intense stirring by means of an Ultra-Turrax® T5 mixer operating at 12 000 rpm for 2 min, at 20 °C. The primary $W_1$/O emulsions were dispersed at 30 wt.% in an aqueous phase containing 12 wt.% NaCAS and 0.8 mol.L$^{-1}$ D-glucose using the Couette's cell. The average diameters of the aqueous droplets were close to 2-3 $\mu$m and that of the oil globules was around 25 $\mu$m. Immediately after fabrication, the emulsions were submitted to a 10-fold dilution (by wt.) with either pure water or an iso-osmotic solution. Due to the large osmotic pressure mismatch, an osmotic swelling phenomenon was expected to occur for the emulsions diluted with pure water, consisting in the transfer of water from the external $W_2$ phase into the inner $W_1$ droplets through the oil phase.

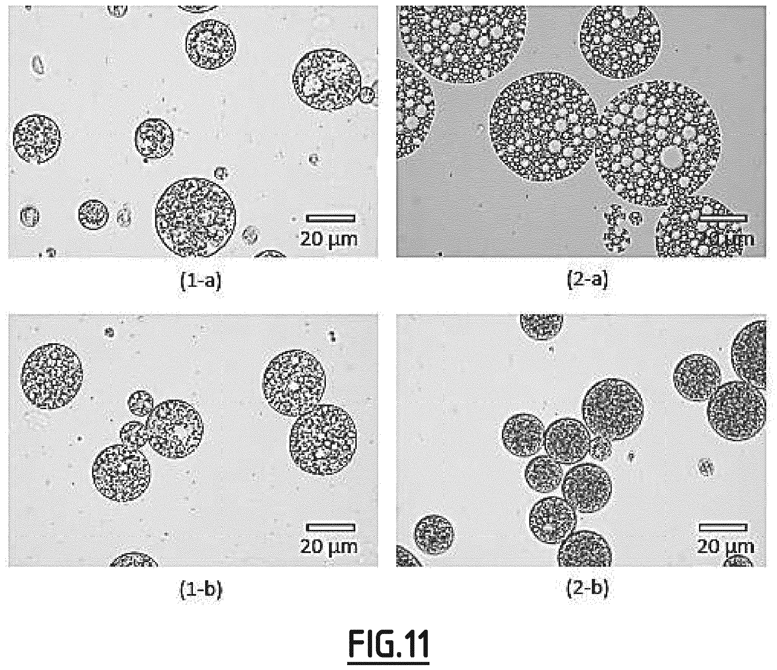

The macroscopic aspect of the double emulsions after 2 hours of settling at 4 °C was evaluated. The globules form a cream layer sitting at the top of the tubes. A thin creamed layer is formed for emulsion 1, irrespective of the diluting conditions, reflecting the absence of water transfer. The height of the creamed layer in emulsion 2 is considerably larger for the system diluted with pure water, due to the swelling phenomenon. Under iso-osmotic conditions, the thickness of the cream layer is comparable to that of emulsion 1. The diluted emulsions were observed under the microscope after 2h-storage. Fig 11 reveals the absence of swelling for emulsion 1, whatever the conditions, and for emulsion 2 diluted under iso-osmotic conditions. Conversely, the globules of emulsion 2 diluted with pure water are noticeably larger than the initial ones and contain large tiny packed inner droplets, evidencing water transfer.

From the experiments, it is obvious that the swelling process is fast for emulsions containing liquid oil and PGPR and is almost undiscernible in presence of fat crystals.

For emulsion 2, swelling was not discernable even after 7 days of storage at 4 °C. It was demonstrated that PGPR-based emulsions were sensitive to the presence of a salt concentration gradient, whereas fat crystal-stabilized emulsions according to the invention showed little response.

According to the invention, fat crystals around W1 droplets are considered to form a thick and continuous solid layer hindering the formation of thin liquid films for the diffusive transport of water. This property makes double emulsions based on crystallized oil remarkably resistant to osmotic shocks.

**3.6. Probing the generality of the approach**

The generality of the scope of the invention was proved by replacing AMF by fats from various vegetal sources: i.e. for example cocoa, palm and coconut.

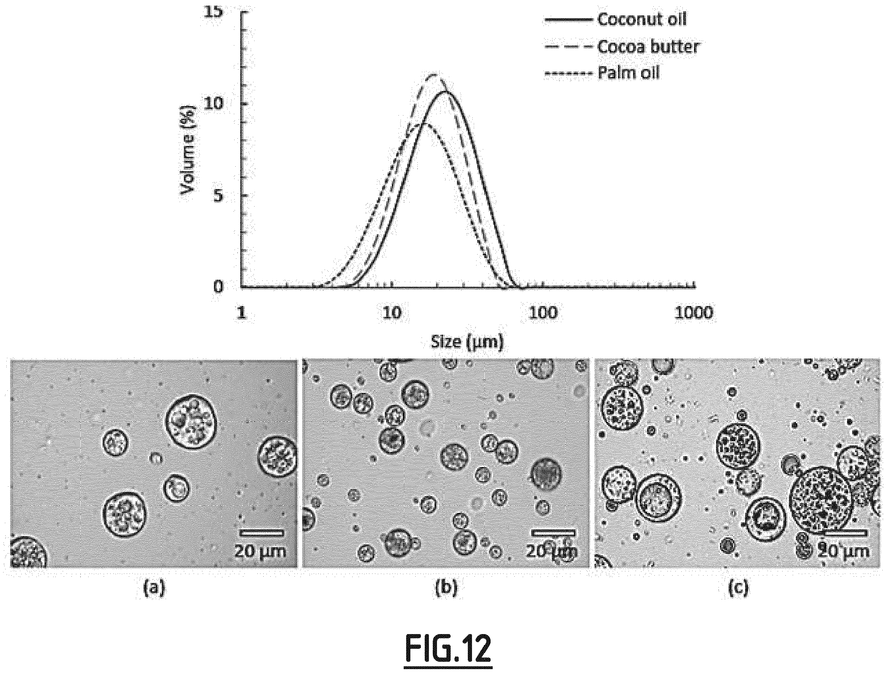

In all cases the emulsions had the following initial composition: 30 wt.% globules; $\phi_i^0$ = 30 wt.%; 0.5 mol.L$^{-1}$ NaCl in W$_1$; 12 wt.% NaCAS and 0.8 mol.L$^{-1}$ D-glucose in W$_2$. They were obtained following the protocol described in example 1, and the applied shear rate during the second emulsification step was 10,500 s$^{-1}$. Fig. 12 (a,b,c) shows microscopic images of the double emulsions right after fabrication. The corresponding size distributions measured by static light-scattering were also reported. The size distributions were relatively narrow in all cases, especially for the double emulsions based on cocoa butter. From the images it can be stated that a significant fraction of the inner droplets remained encapsulated in the globules.

Quasi-monodisperse double emulsions of the W$_1$/O/W$_2$ type, without adding any lipophilic surfactant were designed. In the primary W$_1$/O emulsion, the aqueous droplets were stabilized by the fat crystals forming an oleogel. The process is rather versatile and could be reproduced with fats from several sources including milk, palm, cocoa and coconut.

The obtained multiple emulsions have a widespread application potential including the encapsulation of hydrophilic drugs and their controlled delivery, the implementation of taste masking strategies, the preparation of low-fat products, etc. The average globule size could be finely tuned through the application of a controlled shear and the size distributions were remarkably narrow. Monodispersity is a valuable property since it enables fabrication of materials with reproducible and well-characterized properties. In addition, the obtained materials exhibited outstanding properties: they were thermally sensitive, they withstood osmotic shocks and they were not subject to partial coalescence under storage conditions despite the presence of fat crystals.

**Example 4 - Preparing a double emulsion of the A/O/W type according to the invention (A/O/W2)**

**- Whipping of the crystallized oil phase and bubble refining**

AMF (anhydrous milk fat provided by Barry Callebaut (Belgium)) initially stored at 4°C for several weeks was warmed up to 20 °C and sheared using an Ultra-Turrax® T5 mixer operating at 24,000 rpm for 30 seconds. Then, 50 g of sheared AMF were introduced into a 100 ml beaker and whipped for 5 min using an IKA RW20 overhead stirrer equipped with 4-bladed R 1342 propeller, at a maximum rotation rate of 2,000 rpm. With the aim of reducing the bubble size, the obtained foam was sheared within a narrow gap in a Couette cell (TSR, France; concentric cylinders' configuration) at 20 °C. The inner cylinder of radius r = 20 mm was moved by a motor that rotates at a selected angular velocity, $\omega$, which can reach up to 78.5 rad.s-1. The outer cylinder was immobile, and the gap between the stator and the rotor was fixed at e = 200 $\mu$m. For the maximum angular velocity, high shear rates could be obtained, namely $\dot{\gamma}$ = r$\omega$/e = 7,850 s-1, in simple shear flow conditions.

**Characterization of the oil foam**

The overrun of the oil foam is defined as the volume percent of air incorporated in the oil phase. To measure it, approximately 15 mL of foam were introduced in a graduated Falcon® tube and the initial level was marked. The sample was then centrifuged at 11,700 g, g being the earth gravity constant, for 15 min at 50 °C. AMF was fully molten at this temperature, allowing fast creaming of the air bubbles and their release at the top of the tube. The sample was finally cooled for 2 h at room temperature to allow recrystallization of AMF. The volume variation was divided by the initial volume to obtain the overrun.

The size distributions of air bubbles were estimated by image analysis. Images were obtained using an Olympus BX51 microscope (Olympus, Germany) equipped with an oil immersion objective and a digital camera (Olympus U-CMAD3, Germany). The dimensions of about 350 droplets were measured to determine the volume-averaged diameter, D [4;3], and polydispersity, U, defined in Eq.(1). The measured diameters were subdivided into 20 granulometric classes.

**Fabrication of multiple A/O/W emulsions**

Right after the formation of the oil foam, coarse multiple A/O/W emulsions were prepared by incorporating the foam at 20 wt.% in an external aqueous phase under manual stirring. In dilute emulsions, for droplet deformation and break-up to occur, the applied shear stress, $\eta_c\dot{\gamma}$, where $\eta_c$ is the viscosity of the continuous phase and $\dot{y}$ is the applied shear rate, must be sufficiently high. In order to fragment the drops at relatively low shear rates (laminar regime), large viscosities are required. In the invention's experiments, this was achieved by dissolving a large amount of proteins in the continuous phase. The external phase was composed of 12 wt.% NaCAS. Multiple emulsions were then obtained by submitting the coarse emulsion to different shear rates in the aforementioned Couette cell, at 20 °C. The obtained multiple emulsions were finally submitted to a two-fold dilution in a gelled solution containing 10 wt.% hydroxyethyl cellulose, before storage at 4 °C.

**Multiple emulsions characterization**

The droplet size distribution of A/O/W emulsions was measured using a Mastersizer 2000 Hydro SM. Measurements were performed directly after emulsification. Static light scattering data were transformed into size distribution using Fraunhofer theory, valid for droplets whose size exceeds 5-10 $\mu$m. The A/O/W emulsion (1 mL) was diluted in 10 mL of a SDS solution at $8 \times 10^{-3}$ mol.L$^{-1}$. A small volume of sample was then introduced under stirring in the dispersion unit containing a solution of Tween® 80 at $1.2 \times 10^{-5}$ mol.L$^{-1}$ to avoid foaming and droplet deposition on the optics. The obtained results were systematically checked using optical microscopy. The emulsions were characterized in terms of their volume-averaged diameter, $D$ [4;3], defined as in Eq. (1).

The encapsulation yield of air is defined as the percent of air remaining in the oil globules after the emulsification step, relative to the volume of air in the primary A/O foam. The fraction of air in A/O/W emulsions was estimated by means of the following procedure. A volume $V_1$ of the emulsions was introduced in 15 mL Falcon® tubes and was centrifuged at 11 500 g for 15 min at 50 °C. The largest globules coalesced and formed a macroscopic oil phase residing at the top of the tubes. The encapsulated air was readily released from it and, in the absence of air bubbles, the molten oil phase became transparent. A thin creamed layer made of non-coalesced droplets was observed underneath the oil phase. Observations under the microscope revealed that these droplets still contained air bubbles. This is why the tubes were introduced in a vacuum bell jar and depressurized down to 0,5 bar. This treatment resulted in full delivery of the air bubbles. After this process, the new volume, $V_2$, was measured. The encapsulation yield was then straightforwardly obtained:

$$Encapsulation\ yield = \frac{V_1 - V_2}{V_{Air}^0} = \frac{V_1 - V_2}{V_{Foam \times Overrun}} \qquad (4)$$

where $V_{Foam}$ is the foam volume initially introduced in the double emulsion. The measurement of its overrun was made according to the protocol defined above.

**Results**

**Whipping of AMF**

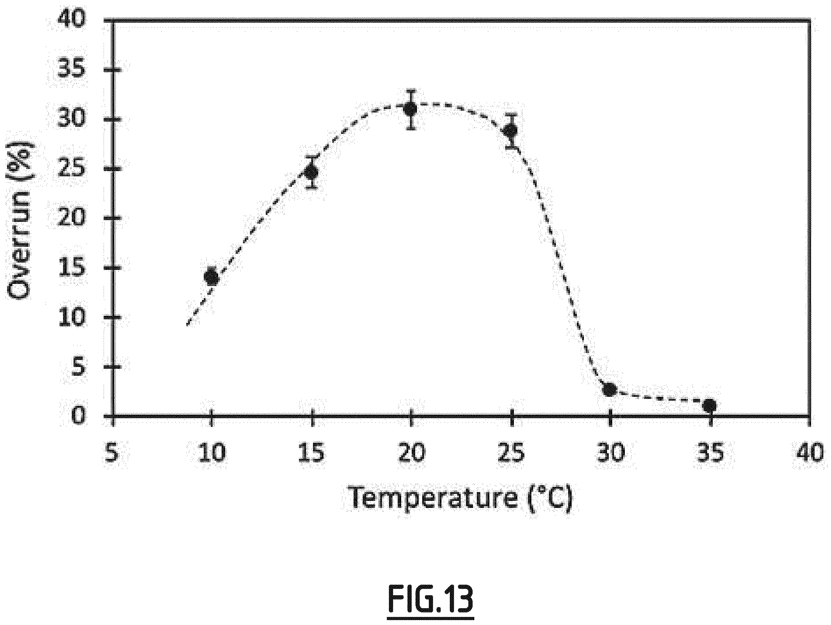

Optimal conditions allowing to aerate AMF in terms of stirring conditions and temperature were first determined. It turned out that the turbulent shear applied by rotor-stator devices like Ultra-Turrax® provided low overrun levels (<20%). The very high shear rates and the small rotating head were not suited for the incorporation of the air bubbles in the sample. This is why a propeller type device was adopted as well as a relatively moderate rotating rate, namely 2,000 rpm. Figure 13 displays the variation of the overrun measured after 5 min of stirring as a function of temperature. The evolution is not monotonous, with the overrun being noticeably smaller at 10 °C and 30 °C than at 20 °C. The presence of a peak in the plot confirms the existence of an optimum solid fat content for foaming. In the case of AMF, the optimum temperature of 20 °C corresponds to a solid fat content of about 15-20% (F. Thivilliers, E. Laurichesse, H. Saadaoui, F.L. Calderon, V. Schmitt, Thermally induced gelling of oil-in-water emulsions comprising partially crystallized droplets: The impact of interfacial crystals, Langmuir. 24, 13364-13375 (2008)). Below 20 °C the viscosity of the oil phase increased considerably, which made air incorporation more difficult. At 30 °C and above, the crystals' content was insufficient (< 10%) (Thivilliers et al. (2008) as mentioned above) to ensure stabilization of the air bubbles and the overrun decayed dramatically. As the maximum overrun was obtained at 20 °C, this temperature was selected for all subsequent experiments.

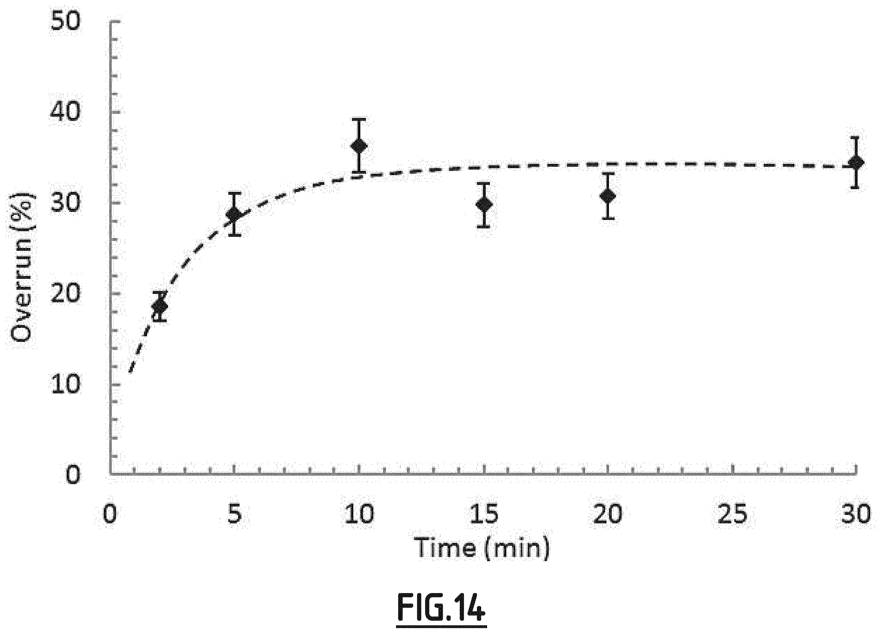

The agitation time was varied between 0 and 30 min at 20 °C in order to probe the kinetics of the whipping process. The evolution of the overrun as a function of time in reported in Figure 14. Foams containing around 30-35 % air were obtained by whipping AMF for 5 min and no further evolution of the overrun was noticed when the shearing time was prolonged.

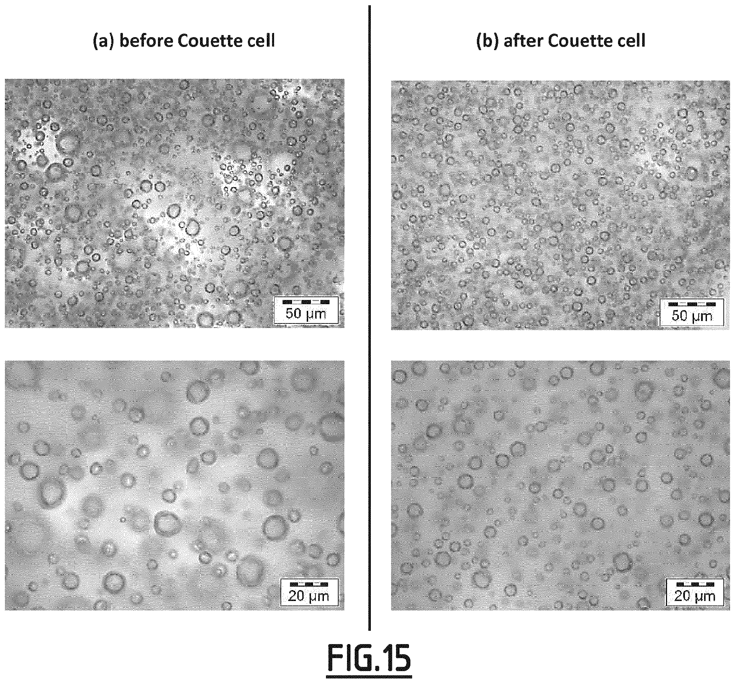

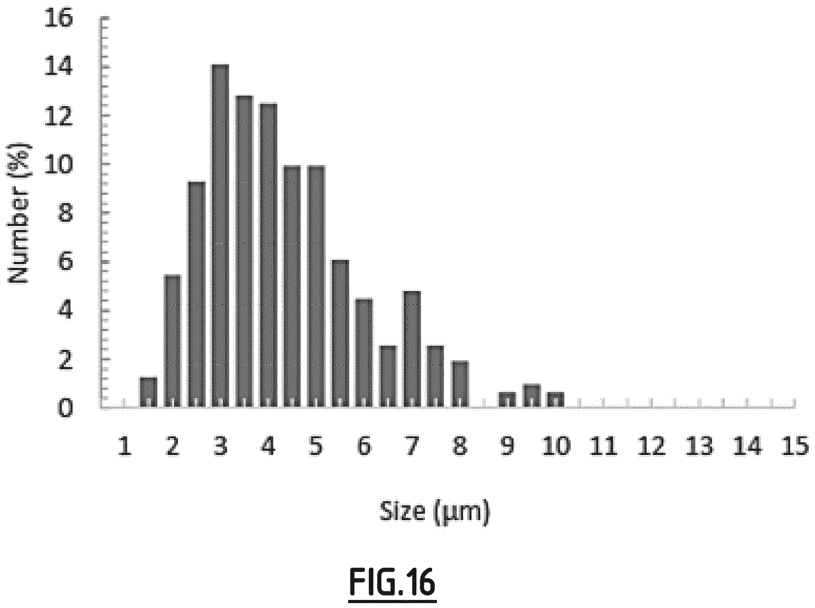

According to microscope images of an oil foam obtained after 5 min of whipping at 20°C, the volume averaged bubble size is close to 20 $\mu$m. Observations were also made at longer whipping times and the average size did not evolve. The objective being to obtain A/O/W emulsions whose globule diameter does not exceed several tens of micrometers, it is important to reduce the bubble size as much as possible. In order to refine it, the coarse foam was sheared at 5,250 s$^{-1}$ in a Couette cell at 20 °C. Figure 15 (right column) shows characteristic micrographs after the refining step. The comparison of the images reveals the added value of processing the foam in the Couette cell: the size distribution of the bubble was sharpened and their average droplet size was reduced. Moreover, the refining step did not vary the overrun which remained close to 30% as long as the applied shear rate was lower than about 6,000 s$^{-1}$. The final air bubbles had a nearly spheroidal shape and their surface was rough. They were stable against coalescence and Oswald ripening for several weeks when stored at 4 °C. It is likely that crystals formed a jammed layer around the bubbles, providing a Pickering-type stabilization. Their size distribution is reported in Figure 16. The volume-averaged bubble size (D[4,3]) was 6.4 $\mu$m and the polydispersity was $U$= 0.22%.

**Dispersion of the whipped oil in an aqueous phase**

Emulsification of the oil foam in the external aqueous phase was always carried out less than 30 min after fabrication of the foam to avoid any structural evolution of the fat crystals (aggregation and/or sintering) that would produce fat hardening. In these conditions, the viscosity of the non-aqueous foam was low enough to process the material under laminar flow conditions.

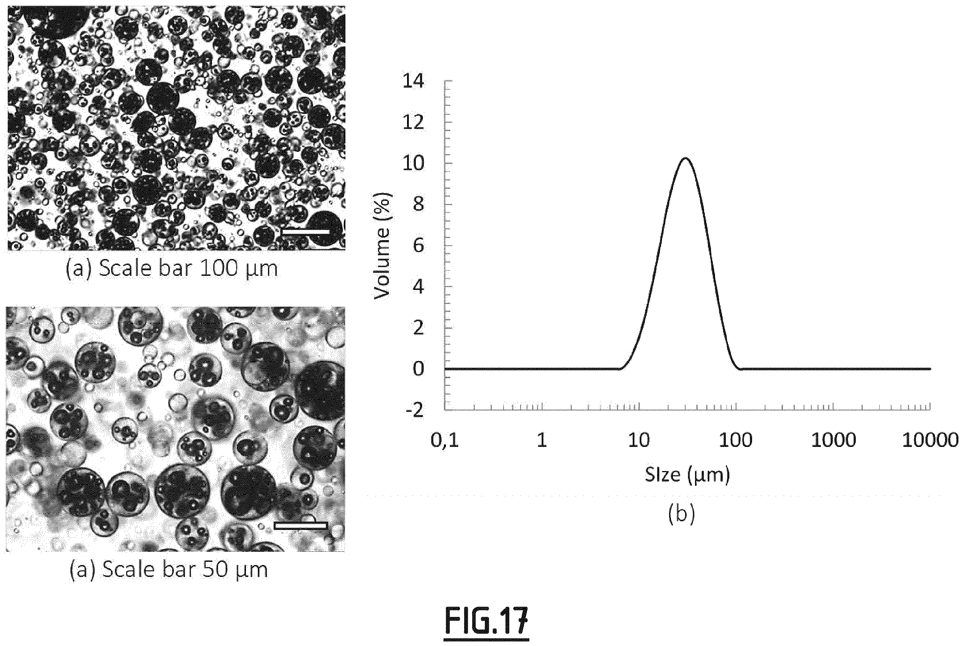

The non-aqueous foams, initially containing about 30 vol.% air, were dispersed at 4 °C in a highly viscous aqueous phase with 12 wt.% NaCAS. The oil foam was progressively introduced in the aqueous phase up to 20 wt.% under manual stirring. The obtained coarse emulsion was then sheared in a Couette cell at 20 °C to obtain the final multiple A/O/W emulsion. The applied shear was varied between 1,050 and 7,350 s$^{-1}$. Figure 17 shows micrograph of the multiple emulsion obtained after applying a shear rate of 3,150 s$^{-1}$ and the corresponding size distribution. The average globule diameter was 30 $\mu$m. The emulsion exhibited a narrow size distribution, as reflected by the relatively low value of the polydispersity index: $U$= 0.39. The presence of air bubbles within the oil globules is clearly evidenced owing to the large refractive index mismatch between air and AMF. Not surprisingly, most of the air bubbles are confined within the largest globules, whereas small globules (<1 $\mu$m) are frequently devoid of bubbles. At low magnification, large globules appear as dark spheres because light is scattered multiple times by the inner bubbles and, as a result, light is hardly transmitted.

**Influence of the shear rate**

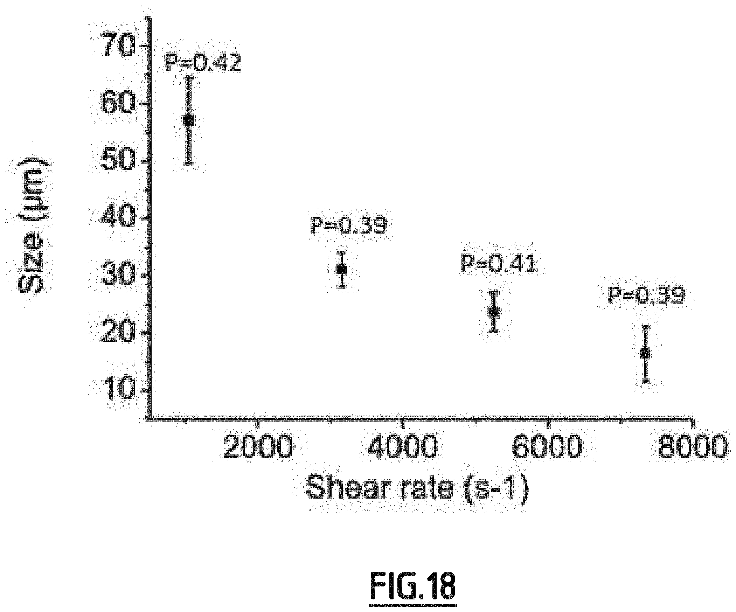

Figure 18 shows the evolution of the mean size characteristics of the multiple globules. The average globule diameter, $D_G$, is plotted as a function of the shear rate, $\dot{y}$, and the polydispersity index, U, is indicated nearby each experimental point.

As expected, increasing the applied shear rate resulted in a reduction of globules size. The experiment was repeated at least 4 times for each shear rate.

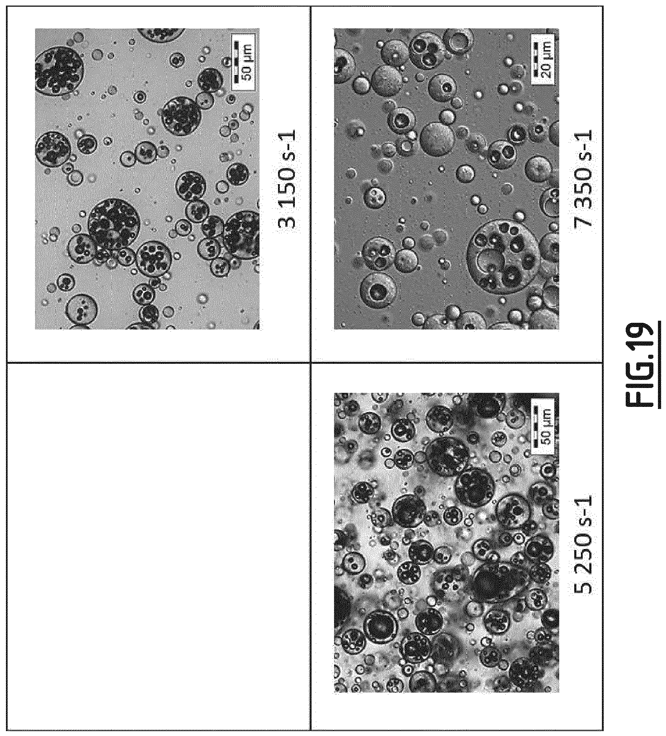

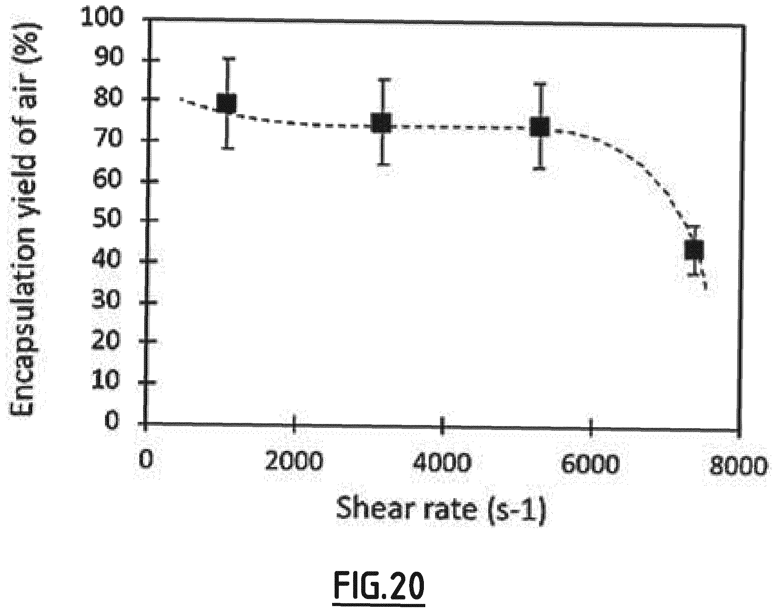

The applied fragmentation method not only successfully produces compartmented globules with a relatively narrow size distribution, but also allows a fine tuning of the average globule diameter by varying the shear rate. Figure 19 shows typical micrographs of multiple emulsion globules obtained at different shear rates. The inner bubble concentration does not seem to vary significantly from 1,050 to 5,250 s$^{-1}$. However, a significant decrease can be observed at 7,350 s$^{-1}$. This tendency was confirmed by performing measurements of the air encapsulation yield (EY) using the protocol previously described. The results are reported in Figure 20. The EY tends to decrease with the applied shear rate, reflecting partial delivery of the air bubbles induced by globules' break up. Similar trends have been frequently reported in W/O/W emulsions. Indeed, it has been observed that release of the inner droplets occurred in conjunction with globule fragmentation. In our systems, the EY remained at a relatively high level, above 74% for shear rates not exceeding 6,000 s$^{-1}$. In this case, globules in the freshly formed emulsion contained 22 % air. Above this threshold shear rate, the applied stress became clearly detrimental to the persistence of the multiple structure, as reflected by the relatively low EY measured at 7,350 s$^{-1}$ (44%) corresponding to 13% air within the globules.

**Stability assessment**

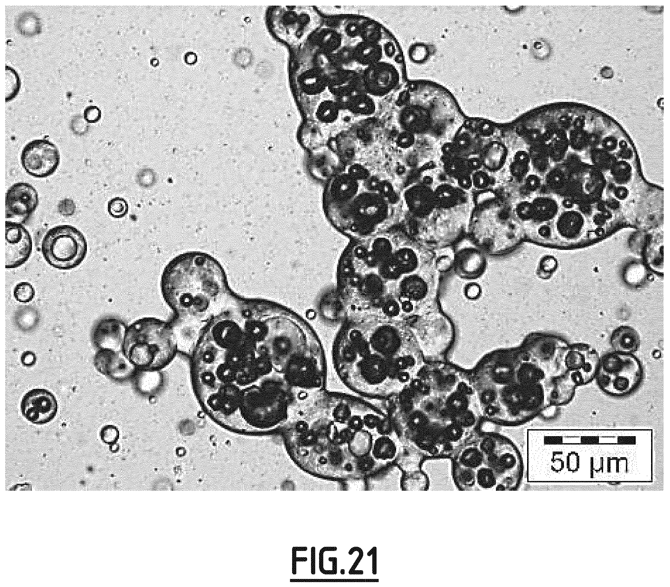

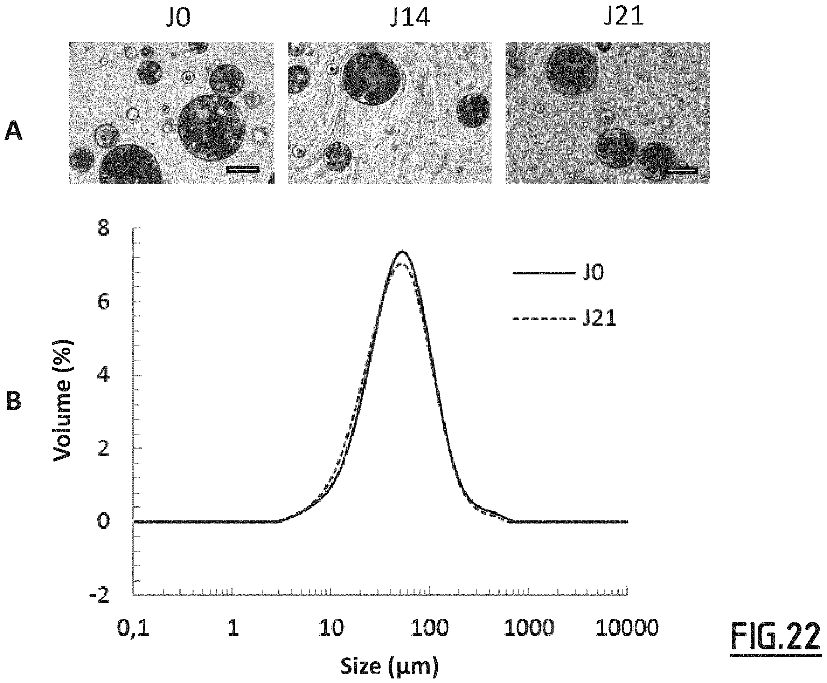

The stability of the obtained emulsions was assessed after prolonged storage at 4 °C. Despite the high viscosity of the aqueous phase, the globules tended to cream at the top of the recipient within a short period of time (several days) because of the significant density mismatch between the air-containing globules and the aqueous phase ($\Delta\rho$ >0,25 g.cm$^{-3}$). In the creamed layer, globules were in permanent contact and this situation provoked partial coalescence, as revealed by Figure 21. To obtain this micrograph, a small amount of cream was collected at the top of the sample and was dissolved in pure water. The micrograph reveals the presence of large aggregates made of remnants of the initial droplets which have coalesced and partially relaxed their shape. When fat crystals are formed nearby the interface, they can protrude into the continuous phase and pierce the thin film between adjacent droplets. This phenomenon is referred to as partial coalescence since the shape relaxation process driven by surface tension is frustrated by the intrinsic rigidity of the partially solidified droplets. In our case, partial coalescence was also favored by the large size of the droplets. To avoid buoyancy-driven instabilities, the emulsions were submitted to a 1:1 w/w dilution with a gelled solution containing 10 wt.% hydroxyethylcellulose. The dilution was carried out right after the emulsification process. The gelled state of the aqueous phase prevented creaming, allowing the system to remain homogeneous for at least 1 month at 4 °C. As a matter of fact, neither the internal structure of the globules nor their average size exhibited any apparent evolution after a storage period of 4 weeks, as revealed by Figure 22.

Emulsions generally exhibit two types of instabilities, coalescence and Ostwald ripening (OR). In the case of multiple A/O/W emulsions, coalescence may occur at different levels: (1) between oil globules, (2) between air bubbles, and (3) between the bubbles and the globule surface. Coalescence between the oil globules and between the air bubbles was inhibited essentially because of the gelled state of the respective phases the objects were embedded in. The air bubbles were physically trapped in an oleogel, *i.e.* a network made of fat crystal aggregates dispersed in liquid oil. The absence of dynamics precluded direct contacts to develop over time, thus allowing to maintain the foamed structure within the oil globules.

The other instability is OR which may potentially occur between the oil globules and between the air bubbles. This instability consists in the molecular diffusion from the smaller to the bigger colloidal objects, due to differences in Laplace pressure. We did not observe OR of air bubbles either probably because of the formation of a rigid layer at the interface which mechanically hindered volume variations of the air bubbles. For the same reason, OR between the air bubbles was not observed in the oil globules, within the time scale of the observations.

## Example 5 - Scale up of a double emulsion of $W_1/O/W_2$ type for fresh dairy applications

The technical feasibility of producing a double emulsion of $W_1/O/W_2$ type into a stirred yogurt was performed at a pilot plant scale using formulations & processing conditions representative of a commercial fermented dairy product (typical batch size of 70 liters).

In total, 3 formulas were prepared by mixing ingredients in stainless steel containers during 10mins at 8°C using a Rushton turbine impeller, to target a constant protein level of 68g/kg (see table below for more details on the ingredients used):

- Formula 1 (F1): with 3 % fat from AMF introduced at the batching stage was taken as a reference without double emulsions,
- Formula 2 (F2) with 3% fat from the addition of 4,3% $W_1/O$ emulsion containing 30% water phase after fermentation,
- Formula 3 (F3) with 2% fat from the addition of 2.86% $W_1/O$ emulsion containing 30% water phase after fermentation.

For F2, the fat content was mainly given by the water-in-oil emulsion, whereas for F3, 33% of the final fat content was already added in the protein mix, through the addition of dairy cream containing 42% fat. This variation in recipe composition induced 2 levels of viscosities for the associated gels after fermentation.

| Target & Ingredients | F1 | F2 | F3 |
|---|---|---|---|
| Target Protein content (g/kg) | 68 | 68 | 68 |
| Target Fat content (g/kg) | 30 | 30 | 30 |
| Skimmed milk (kg) | 60.24 | 62.62 | 60.92 |
| Dairy Cream (kg) | - | - | 1,61 |
| Skimmed milk powder (g/kg) | 7.66 | 7.38 | 7.47 |
| AMF, melting point 32°C (kg) | 2.1 | - | - |
| $W_1/0$ emulsion (%), after fermentation | - | 4.3 | 2.86 |

.For the F1 formula, AMF initially stored at 4°C, was melted in a water bath at 80 °C, before being added to the protein mix using a Liquiverter. To obtain a homogeneous recipe, the F1 formula was then homogenized at 70 bars to emulsify AMF in the protein mix. All the formulas were then stored at 4°C before pasteurization.

During the pasteurization, the homogenizer was used in upstream position with the following parameters:

- Homogenization pressure of 163 bar
- Pasteurization temperature 95 °C
- Holding time 6 min

- Flow rate 250Uh
- Outlet temperature 29 °C.

The formulas were then stored in an incubator at 28 °C before inoculation with lactic acid bacteria.

The fermentation was performed using lyophilized lactic acid bacteria culture containing *Streptococcus thermophilus and Lactobacillus bulgaricus*. A batch of 50 DCU was dispersed in 500ml of the protein mix from the F1 formula. Each formula was then inoculated with this solution until a pH 4.60 or less was obtained.

The F1 formula was first manually mixed before being transferred into a storage tank at a flow rate of 300Uh and sheared at 50 Hz or 1,500RPM with an inline rotor/stator device of 130mm in external diameter, composed of 3 toothed cages per rotor & stator with a slot width of 3mm, and then cooled at 4 °C thanks to a plate heat exchanger.

Once the F1 formula was sheared, it was stored at 4 °C. A quick sensory test evidenced a strong protein/powder taste, due to the high content of SMP.

The $W_1/0$ emulsion was then prepared for injection in the formulas F2 and F3. To prepare a 5 kg-batch of water-in-oil emulsion, AMF was melted in a water bath at 80 °C with water and salt. The mixture with the following composition was then pasteurized using an industrial batch cooker equipped with a scrapper and a blade-type impeller (RoboQbo):

- 3.50 kg AMF
- 1.50 kg (1.460 kg water+ 0.040 kg salt)

The parameters used during the pasteurization were as follows: heating at 92 °C for 60 s, cooling to 30 °C for 1,800 s, stirring with a blade at 50 rpm during the whole process. The pasteurized ingredients were then stored at 30 °C under continuous stirring before emulsification. The pasteurized AMF/water blend was emulsified using a 2 stages bench homogenizer. First, the homogenizer was cleaned and warmed with hot water. Then the AMF/water blend was homogenized using a homogenization pressure of 20/80 bar (2 stages). The color of the obtained water-in-oil emulsion evolved from yellow to white during emulsification suggesting the formation of the $W_1/O$ emulsion.

Some batches of water-in-oil emulsion were homogenized 2 times to improve the homogeneity. The final water-in-oil emulsion was weighed for each formula and stored in an incubator at a temperature range of 25-30 °C.

Each emulsion was manually added to the F2 and F3 formulas. Two intensities of shearing using the inline rotor/stator device (corresponding to 50 & 100Hz respectively) were tested. The size of the $W_1/O$ emulsion was measured thanks to static light scattering experiments. The results showed that the emulsion obtained had a narrower size distribution with an average diameter of 20 $\mu$m when the highest frequency was used. This frequency was then kept constant.

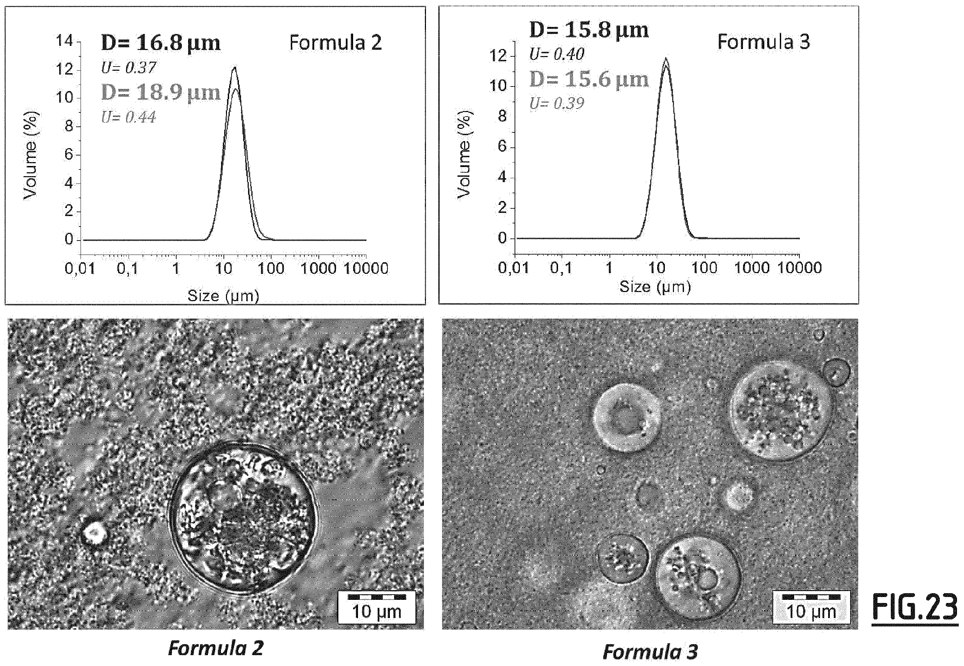

After shearing with the inline rotor/stator device, the obtained yogurts were stored at 4 °C in a cold chamber. Macroscopically, no differences were observed between the yogurt containing multiple emulsions and the references. The yogurts with a double emulsion structure were smooth, stable with less astringent perception. Microscopic analyses were finally performed at D+14 days after the production on the F2 & F3 formulas in order to validate the presence of internal aqueous droplets in the fat globules. Size measurements were performed using a Mastersizer 3000, the average diameter of the fat droplets was around 20 $\mu$m (Figure 23).

## Claims

1. A multiple emulsion comprising or consisting of P1/O/W2, wherein P1 is an aqueous phase forming droplets or a gas phase forming bubbles, said droplets or bubbles being dispersed in O thereby forming P1/O, wherein O is an oily phase comprising crystals, wherein W2 is an aqueous phase comprising at least one hydrophilic surfactant, wherein P1/O globules are formed in W2, wherein said oily phase O comprises at least 90%, preferably at least 92%, and even preferably at least 95%, by mass of triglycerides with respect to the mass of the O phase.

2. The multiple emulsion according to claim 1, wherein P1 is a gas phase is air or a gas comprising nitrogen.

3. The multiple emulsion according to claim 1, wherein the average diameter of P1 aqueous droplets ranges from 1 $\mu$m to 10 $\mu$m, preferably from 1 $\mu$m to 5 $\mu$m.

4. The multiple emulsion according to claim 1, wherein the average diameter of P1 gas bubbles ranges from 5 $\mu$m to 20 $\mu$m.

5. The multiple emulsion according to claim 1, wherein the average diameter of the fat globules ranges from 5 $\mu$m to 100 $\mu$m.

**6.** The multiple emulsion according to claim 1, wherein P1 comprises a hydrophilic active ingredient, for example selected from the group consisting of hydrophilic pharmaceutically or cosmetic active ingredients, hydrophilic sensory active ingredients, and any combination thereof.

**7.** The multiple emulsion according to claim 1, wherein O comprises from 5 to 60%, preferably, less than 40 % by mass of P1 relative to the total mass of P1/O phases.

**8.** The multiple emulsion according to claim 1, wherein A represents a fraction ranging from 20 to 40%, the % being expressed in volume of gas with respect to the total A/O foam volume.

**9.** The multiple emulsion according to claim 1, wherein said multiple emulsion comprises from 5 to 60%, preferably, from 10 to 40 % by mass of P1/O phases relative to the total mass of multiple emulsion.

**10.** The multiple emulsion according to claim 1, wherein O comprises or consists of one or more edible fats, for example selected from the group consisting of butter, milk fat, anhydrous milk fat, tallow, lard, mutton fat, poultry fat, fish oil, cocoa butter, palm oil, coconut oil, tree-nut oil, legume oil, sunflower oil, safflower oil, corn oil, cottonseed oil, soybean oil, canola oil, peanut oil, palm oil, palm olein, palm super-olein, palm kernel oil, algae oil, flaxseed oil, or any combination thereof.

**11.** The multiple emulsion according to claim 1, wherein W2 has a dynamic viscosity ranging from 0.1 to 1 Pa.s.

**12.** A method for preparing a multiple emulsion, said multiple emulsion being represented by P1/O/W2, wherein P1 is an aqueous phase forming droplets or a gas phase forming bubbles, said droplets or bubbles being dispersed in O thereby forming P1/O, wherein O is an oily phase comprising crystals, and W2 is an aqueous phase, and W2 is identical or different than P1, wherein said composition comprise less than 5% by mass of endogenous lipophilic surfactants with respect to the total mass of the multiple emulsion, wherein said process comprising the following steps:

- dispersing P1 in O, at a temperature where O contains crystals, to obtain a primary P1/O double phase; advantageously said crystals stabilize the primary P1/O double phase,
- dispersing ,P1/O in W2 at a temperature where O contains crystals, and wherein W2 contains one or more proteins to obtain a P1/O/W2 multiple emulsion.

**13.** The method according to claim 11, wherein said P1/O/W2 multiple emulsion is stored under conditions wherein O contains crystals.

**14.** The method according to claim 11 or 12, wherein the dynamic viscosity of P1/O system is low enough to process the material under laminar flow conditions.

**15.** The method according to any one of claims 11 to 13, wherein the shear rate for dispersing P1/O in W2 is ranging from 1,000 to10,000 s$^{-1}$.

**16.** A composition, preferably a food, a cosmetic or a pharmaceutical composition comprising a multiple emulsion as defined in any one of claims 1 to 10 or as obtainable by a method as defined in any one of claims 11 to 14.

**17.** A method for preparing composition, preferably a food, a cosmetic or a pharmaceutical composition, said method comprising incorporating a multiple emulsion as defined in any one of claims 1 to 10 or as obtainable by a method as defined in any one of claims 11 to 14 into a base composition to form said composition.

**18.** A packaging containing a composition as defined claim 15 or as obtainable by a method as defined in claim 16.

FIG.1

FIG.2

FIG.3

FIG.4

FIG.5

**FIG.6**

FIG.7

FIG.8

FIG.9

FIG.10

(1-a)

(2-a)

(1-b)

(2-b)

## FIG.11

FIG.12

FIG.13

**FIG.14**

(a) before Couette cell    (b) after Couette cell

FIG.15

FIG.16

FIG.17

FIG.18

FIG.19

**FIG.20**

FIG.21

FIG.22

*Formula 2*                    *Formula 3*

FIG.23

EP 3 662 756 A1

EP 3 662 756 A1

# EUROPEAN SEARCH REPORT

**Europäisches Patentamt / European Patent Office / Office européen des brevets**

Application Number
EP 18 30 6648

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | GERALD MUSCHIOLIK ET AL: "Double Emulsions Relevant to Food Systems: Preparation, Stability, and Applications : Double emulsions in food...", COMPREHENSIVE REVIEWS IN FOOD SCIENCE AND FOOD SAFETY, vol. 16, no. 3, 23 March 2017 (2017-03-23), pages 532-555, XP055583318, US ISSN: 1541-4337, DOI: 10.1111/1541-4337.12261 * table 1 * * "Systems of the W/O/W type...."; page 532, right-hand column, paragraph 2 * * "The standard procedure..."; page 533, left-hand column, paragraph 2 * * "W/O/W emulsions"; page 535, left-hand column - right-hand column * | 1-18 | INV. A23D7/015 A23C9/12 A23G9/40 A23L35/00 ADD. A61K8/06 B01F3/08 |
| T | MATHIEU BRUN ET AL: "Stabilization of air bubbles in oil by surfactant crystals: A route to produce air-in-oil foams and air-in-oil-in-water emulsions", FOOD RESEARCH INTERNATIONAL, vol. 67, 1 January 2015 (2015-01-01), pages 366-375, XP055583484, AMSTERDAM, NL ISSN: 0963-9969, DOI: 10.1016/j.foodres.2014.11.044 * page 367, paragraph 2.6 * * page 373, paragraph 3.3 * * paragraph [0004] * | 1-16 | TECHNICAL FIELDS SEARCHED (IPC) A61Q A61K B01F A23D C11C A23C A23G A23L |

-/--

The present search report has been drawn up for all claims

| Place of search | Date of completion of the search | Examiner |
|---|---|---|
| Munich | 26 April 2019 | Oenhausen, Claudia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

Application Number

EP 18 30 6648

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2011/077073 A1 (UNIV BIRMINGHAM [GB]; SPYROPOULIS FOTIS [GB] ET AL.) 30 June 2011 (2011-06-30) * No PGPR; page 3 * * page 4, paragraph 4; examples 1-3 * * page 2, paragraph 2 - paragraph 4 * ----- | 1,10,12, 16-18 | |
| X | US 6 627 603 B1 (BIBETTE JEROME [FR] ET AL) 30 September 2003 (2003-09-30) * column 2, line 46 - column 3, line 13 * * column 3, line 46 - line 49 * * column 4, line 63 - line 65 * * column 5, line 11 - line 13 * * column 5, line 22 - line 23 * * column 5, line 31 - line 43 * ----- | 1,10,12, 16-18 | |
| X | EP 3 370 537 A1 (UNILEVER NV [NL]; UNILEVER PLC [GB]) 12 September 2018 (2018-09-12) * paragraph [0001] - paragraph [0003] * * paragraph [0017] - paragraph [0021] * * paragraph [0041] * * paragraph [0076] * * paragraph [0025] * * paragraph [0057] * ----- | 1-18 | **TECHNICAL FIELDS SEARCHED (IPC)** |
| X | EP 2 077 106 A1 (CONTROLLED LIPO TECHS INC [JP]) 8 July 2009 (2009-07-08) * paragraph [0012] - paragraph [0013] * * paragraph [0027] * * paragraph [0035] - paragraph [0036] * ----- | 1,10,12, 16-18 | |
| X | EP 0 174 377 A1 (MEIJI MILK PROD CO LTD [JP]) 19 March 1986 (1986-03-19) * page 1, line 6 - line 12 * * page 6, line 6 - line 25 * * Test results; page 8 - page 17 * ----- | 1,10,12, 16-18 | |

The present search report has been drawn up for all claims

| Place of search | Date of completion of the search | Examiner |
|---|---|---|
| Munich | 26 April 2019 | Oenhausen, Claudia |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 18 30 6648

This annex lists the patent family members relating to the patent documents cited in the above-mentioned European search report.
The members are as contained in the European Patent Office EDP file on
The European Patent Office is in no way liable for these particulars which are merely given for the purpose of information.

26-04-2019

| Patent document cited in search report | | | Publication date | Patent family member(s) | | | Publication date |
|---|---|---|---|---|---|---|---|
| WO 2011077073 | A1 | | 30-06-2011 | NONE | | | |
| US 6627603 | B1 | | 30-09-2003 | AT | 237395 | T | 15-05-2003 |
| | | | | AU | 729461 | B2 | 01-02-2001 |
| | | | | CA | 2267083 | A1 | 18-02-1999 |
| | | | | DE | 69813498 | T2 | 24-12-2003 |
| | | | | EP | 0930933 | A1 | 28-07-1999 |
| | | | | ES | 2200364 | T3 | 01-03-2004 |
| | | | | FR | 2767064 | A1 | 12-02-1999 |
| | | | | JP | 3725559 | B2 | 14-12-2005 |
| | | | | JP | 2000503685 | A | 28-03-2000 |
| | | | | PT | 930933 | E | 31-07-2003 |
| | | | | US | 6627603 | B1 | 30-09-2003 |
| | | | | WO | 9907463 | A1 | 18-02-1999 |
| EP 3370537 | A1 | | 12-09-2018 | AR | 106592 | A1 | 31-01-2018 |
| | | | | BR | 112018009058 | A2 | 30-10-2018 |
| | | | | CA | 3001928 | A1 | 11-05-2017 |
| | | | | CL | 2018001200 | A1 | 20-07-2018 |
| | | | | EA | 201891105 | A1 | 31-10-2018 |
| | | | | EP | 3370537 | A1 | 12-09-2018 |
| | | | | PH | 12018500738 | A1 | 15-10-2018 |
| | | | | US | 2018310583 | A1 | 01-11-2018 |
| | | | | WO | 2017076580 | A1 | 11-05-2017 |
| EP 2077106 | A1 | | 08-07-2009 | CA | 2666534 | A1 | 08-05-2008 |
| | | | | CN | 101547685 | A | 30-09-2009 |
| | | | | EP | 2077106 | A1 | 08-07-2009 |
| | | | | JP | 5196760 | B2 | 15-05-2013 |
| | | | | JP | 2008106020 | A | 08-05-2008 |
| | | | | KR | 20090069327 | A | 30-06-2009 |
| | | | | RU | 2009120058 | A | 10-12-2010 |
| | | | | US | 2010099639 | A1 | 22-04-2010 |
| | | | | WO | 2008053799 | A1 | 08-05-2008 |
| EP 0174377 | A1 | | 19-03-1986 | DE | 3590135 | T1 | 15-05-1986 |
| | | | | EP | 0174377 | A1 | 19-03-1986 |
| | | | | GB | 2165163 | A | 09-04-1986 |
| | | | | JP | S60199833 | A | 09-10-1985 |
| | | | | NL | 8520061 | A | 02-01-1986 |
| | | | | US | 4931210 | A | 05-06-1990 |
| | | | | US | 4971721 | A | 20-11-1990 |
| | | | | US | 4985173 | A | 15-01-1991 |
| | | | | US | 4988456 | A | 29-01-1991 |
| | | | | WO | 8504346 | A1 | 10-10-1985 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **FRASCH-MELNIK, S. ; SPYROPOULOS, F. ; NORTON, I.T.** W1/O/W2 double emulsions stabilized by fat crystals--formulation, stability and salt release. *Journal of Colloid and Interface Science,* 2010, vol. 350 (1), 178-185
- **BRUN M. ; DELAMPLE M. ; HARTE E. ; LECOMTE S. ; LEAL-CALDERON F.** Stabilization of Air Bubbles in Oil by Surfactant Crystals: A Route to Produce Air-in-Oil Foams and Air-in-Oil-in-Water Emulsions. *Food Res. Int.,* 2015, vol. 67, 366-375
- Handbook of Chemistry and Physics. 2017
- **F. THIVILLIERS ; E. LAURICHESSE ; H. SAADAOUI ; F.L. CALDERON ; V. SCHMITT.** Thermally induced gelling of oil-in-water emulsions comprising partially crystallized droplets: The impact of interfacial crystals. *Langmuir,* 2008, vol. 24, 13364-13375